# EUROPEAN PATENT SPECIFICATION

(11) **EP 2 983 720 B1**
(45) Date of publication and mention of the grant of the patent: **01.08.2018**
(21) Application number: 14721226.0
(22) Date of filing: 11.04.2014
(51) Int. Cl.: A61K 47/69, A61K 31/704, A61K 31/164, A61K 31/133, A61K 49/00, A61P 35/00

(54) **PLATFORM FOR TARGETED DELIVERY TO TUMOR CELLS AND USES THEREOF**
PLATTFORM FÜR GEZIELTE ABGABE AN TUMORZELLEN SOWIE DEREN VERWENDUNG
PLATEFORME POUR L'ADMINISTRATION CIBLÉE À DES CELLULES TUMORALES ET SON UTILISATION

(30) Priority: 13.04.2013 PT 2824013
(43) Date of publication of application: 17.02.2016
(73) Proprietor: Universidade de Coimbra, 3004-531 Coimbra (PT)
(72) Inventor: CARVALHO DA FONSECA, Nuno Andre, 3040-450 Coimbra (PT); GOMES DA SILVA, Ligia Catarina, 3000-550 Coimbra (PT); DANTAS NUNES CALDEIRA DE MOURA, Vera Lucia, 3030-502 Coimbra (PT); DE MAGALHAES SIMOES, Sergio Paulo, 3030-329 Coimbra (PT); SERENO DE ALMEIDA MOREIRA, Joao Nuno, 3000-377 Coimbra (PT)
(74) Representative: Bridle, Andrew Barry
(86) International application number: PCT/EP2014/057446
(87) International publication number: WO 2014/167126

(56) References cited:
- WO-A1-2012/101309
- WO-A2-2004/078677
- WO-A2-2006/051549
- WO-A2-2009/088837
- WO-A2-2009/142525
- WO-A2-2010/008876
- WO-A2-2012/154942
- VERA MOURA ET AL: "Targeted and intracellular triggered delivery of therapeutics to cancer cells and the tumor microenvironment: impact on the treatment of breast cancer", BREAST CANCER RESEARCH AND TREATMENT, KLUWER ACADEMIC PUBLISHERS, BO, vol. 133, no. 1, 30 July 2011 (2011-07-30) , pages 61-73, XP035047622, ISSN: 1573-7217, DOI: 10.1007/S10549-011-1688-7 cited in the application
- SHABBITS J A ET AL: "Intracellular delivery of ceramide lipids via liposomes enhances apoptosis in vitro", BIOCHIMICA ET BIOPHYSICA ACTA (BBA) - BIOMEMBRANES, ELSEVIER, AMSTERDAM, NL, vol. 1612, no. 1, 2 May 2003 (2003-05-02), pages 98-106, XP004422679, ISSN: 0005-2736, DOI: 10.1016/S0005-2736(03)00108-1
- WATTERS R J ET AL: "Development and use of ceramide nanoliposomes in cancer", METHODS IN ENZYMOLOGY, vol. 508, 2012, pages 89-108, XP008170674, ISSN: 0076-6879, DOI: 10.1016/B978-0-12-391860-4.00005-7 [retrieved on 2012-03-23]
- GOMES-DA-SILVA LÍGIA C ET AL: "Impact of anti-PLK1siRNA-containing F3-targeted liposomes on the viability of both cancer and endothelial cells", EUROPEAN JOURNAL OF PHARMACEUTICS AND BIOPHARMACEUTICS, vol. 85, no. 3, 7 May 2013 (2013-05-07), pages 356-364, XP028784856, ISSN: 0939-6411, DOI: 10.1016/J.EJPB.2013.04.007

## Description

### FIELD OF THE INVENTION

The invention involves therapy and diagnostics using nanoparticles that provide targeted delivery of agents to cancer cells and stem cells (including cancer stem cells). More specifically, the invention relates to a ligand-targeted delivery system comprising: a targeting ligand that selectively binds a tumor cell, linked to a support, which is a pH sensitive liposome having a cytotoxic ceramide analog encapsulated, entrapped or intercalated in the support, and carrying an agent, which is doxorubicin encapsulated in the support, wherein the molar ratio of cytotoxic ceramide analog to doxorubicin is between 3:1 and 1:2, as defined in the claims.

### BACKGROUND OF THE INVENTION

### Stem Cells

Cell-based therapies are one of the most promising therapeutic paths for human diseases. Amongst them, stem cells play a central role (Mooney,'08; Lodi,'11). Pluripotent stem cells are a population of cells, which possess the potential to differentiate into all the type of cells of the three germ layers: endoderm, mesoderm and ectoderm (De Miguel,'10). Furthermore, stem cells are capable of self-renewal, a characteristic that has allowed their successful isolation and *in vitro* propagation as established immortal cell lines, either from mouse or human blastocysts (Evans,'81; Thomson,'98; De Miguel,'10). Although isolated mouse embryonic stem cells (mESC) and human embryonic stem cells (hESC) share common features, such as gene expression patterns, intrinsically they differ from each other. Amongst the several markers established for identification of pluripotent cells, hESC and mESC express high levels of *Nanog, Oct4* (a.k.a. *POU5F1*), *TDGF* and *GDF3,* which are strongly regulated developmental genes (Adewumi,'07; De Miguel,'10). *Nanog* and *Oct4,* as *Sox2,* are essential regulatory transcription factors for maintenance of self-renewal and undifferentiated status of stem cells. They control several downstream gene targets involved in chromatin remodeling or histone-modifying complexes, correlating with a highly de-condensed chromatin and high nucleus to cytoplasm ratio of stem cells (Thomson,'98; Boyer,'05). Furthermore, these factors also control and inactivate several genes that encode for transcription factors that present major roles in differentiation and development (Boyer,'05). Nonetheless, as mentioned above, differences do exist between mESCs and hESCs. Morphologically, mESC cell colonies are smaller and have a domed shape, whereas those from hESC cells grow as monolayers and are larger (De Miguel,'10). Additionally, cell surface markers such as the glycolipid stage specific embryonic antigens (SSEA) are unequally shared, being the SSEA1 characteristic of mESC cells, whereas hESC cells present SSEA3/4 (Adewumi,'07). Furthermore, the introduction of mouse EpiSC cells (epiblast-derived stem cells) contributed to increase the differences between hESC and mESC (Tesar,'07). These mEpiSC cells share similar features with hESC and mESC cells by expressing the same pluripotency transcription factors, as well as by being able to proliferate continuously and to differentiate into cells from the three initial germ layers (Tesar,'07). However, EpiSC cells are much more similar to hESC cells than to mESC cells, since the latter rely on LiF to maintain pluripotency, which contrasts with the Activin/Nodal pathway required by hESC and EpiSC (Vallier,'09). The similarities between EpiSC and hESC in contrast to mESC, suggested a more differentiated state of hESC when compared to mESC (De Miguel,'10).

Despite all the features mentioned above in stem cell field, additional breakthrough was attained when Takahashi *et al.* demonstrated that it was possible to reprogram somatic cells such as adult fibroblasts, first from mouse and later from human cells, into a state of pluripotency. Upon promoting the expression of four key transcription factors, *Oct4, Sox2, c-Myc* and *Klf4* (Takahashi,'06; Takahashi,'07), induced pluripotent stem cells (iPS) were generated. These human iPS cells closely resembled hESC, showing similar expression patterns of stem cell markers, such as *SSEA3*/*4, Nanog* or *GDF3,* and demonstrating pluripotency capacity by oriented differentiation into neural or cardiac cells (Takahashi,'07). Therefore, by demonstrating similar potential as hESC, iPS cell technology has overcome a limitation of hESC upon enabling the generation of stem cells from patient-derived adult somatic cells, which can be used for individualized cell replacement therapy, thus minimizing cell rejection (Puri,'12).

Nonetheless, stem cells, either isolated from mouse or human embryos, or obtained through iPS technology, present major therapeutic potential due to a wide field of applications, ranging from disease modeling and pharmacological testing, to cell-based therapeutic strategies such as tissue engineering.

### Cancer stem cells

Albeit the therapeutic potential, stem cells may likewise have a role in cancer disease. Tumors are biological entities that can be interpreted as an aberrant dysfunctional organ initiated by a tumorigenic cancer cell with capacity to proliferate indefinitely by acquired mutations (Reya,'01; Visvader,'11). Viewed as an organ, tumors present functional heterogeneity demonstrated by the existence of different populations of cells in the microenvironment. In order to accommodate that functional heterogeneity, a hierarchical organization model of tumor development, known as the cancer stem cell model, was proposed. This model postulates the existence of a sub-population of stem-like cells within the tumor microenvironment, responsible for sustained tumor growth (Reya,'01; Visvader,'08; Visvader,'11). Tumor initiation is not necessarily carried out by that group of cells, but rather by the mentioned tumorigenic cancer cell or cell of origin. That cell might either be a normal stem cell, which has acquired several abnormal transformations, or a partially differentiated cell, like a common progenitor, which has gone through a series of oncogenic hits thus acquiring a stem-like character (Reya,'01; Hanahan,'11). As these cells expand, acquired mutations during neoplastic progression may result in the development of cancer stem cells (CSC), the main responsible for sustained tumor growth and maintenance (Visvader,'11). Despite abnormal, CSC share features of normal stem cells such as self-renewal and differentiation capacity (Reya,'01; Vallier,'09).

The cancer stem cell existence was firstly reported by Bonnet *et al.* (Bonnet,'97) in acute myeloid leukemia, by implicating aberrant hematopoietic cells expressing the CD34⁺/CD38⁻ phenotype (a.k.a. SCID leukemia initiating cells - SL-IC) in disease development, which were also able to differentiate into leukemic blasts. Nevertheless, later on, cancer stem cells were also implicated in solid tumor development by Al-Hajj *et al.* (Al-Hajj,'03). Based on surface markers expression, cells expressing the CD44⁺/CD24⁻/lin⁻ phenotype were isolated from breast cancer patients and implanted in immunocompromised mice, showing increased capacity to develop tumors as well as to be serially passaged and recapitulate the phenotypically diversity of cells present in the primary tumor (Al-Hajj,'03). Additionally, the identification of CSC using alternate markers, such as aldehyde dehydrogenase levels and/or activity, was correlated with poor clinical outcome, metastasis and drug resistance (Ginestier,'07; Croker,'11; Marcato,'11). Direct stem cell involvement in cancer initiation and development was further established in a mouse model of intestinal cancer. Barker and colleagues demonstrated that stem cell specific mutation of the APC gene resulted in the formation of intestinal microadenomas, with sustained hierarchical organization in early lesions (Barker,'09).

### Small molecules for stem cell therapies and cancer treatment

In order to enforce the therapeutic utilization of stem cells in cell-based therapies, they should be converted into functional cells of the tissues or organs to be repaired in a controlled manner. This process, known as directed differentiation, takes advantage of the pluripotent characteristic of stem cells (Cohen,'11; Puri,'12). Directed differentiation can be accomplished by several protocols, either recurring to growth factors or co-culture systems (Cohen,'11). Several growth factors and proteins targeting different signaling pathways have demonstrated to direct stem cell differentiation fate, including Activin A or BMP4, members of the TGFβ family known to direct for endoderm layer (D'Amour,'05). Additionally, FGF family members, such as bFGF, promote retinal differentiation (Lamba,'06). Nonetheless, the use of growth factors or proteins presents some drawbacks since they are normally produced by recombinant technology in engineered bacteria or mammalian cells, often associated with potential source of contamination or limited by the manufacturing-associated cost (Cohen,'11). Therefore, alternatives are required in order to ensure viable application of stem cells in a therapeutic setting.

Increasing attention has been given to small molecules as agents for promoting stem cell oriented differentiation or even somatic cell reprogramming (Cohen,'11; Zhu,'11). Indeed, PD0325901, a MEK inhibitor, has been shown to enhance reprogramming of human fibroblasts (Shi,'08; Zhu,'11). Additionally, the combination of BIX-01294 and BayK8644 compounds has also been shown to promote reprogramming of mouse fibroblasts, in the absence of essential transcription factors (Shi,'08). Nonetheless, directed differentiation of hESC promoted by the inhibitor of the PI3K pathway, LY294002, into endodermal germ line has also been accomplished (McLean,'07). Other compounds have been tested for each application and have been reviewed elsewhere (Cohen,'11; Zhu,'11).

If small molecules are increasingly growing as important tools in stem cell research, they still represent the cornerstone in cancer treatment. However, the drug resistance phenomenon, in which cancer stem cells are major actors, remains as a distressful problem undermining the success of chemotherapy (Visvader,'08; Frank,'10; Visvader,'11). In order to overcome this issue, it has been recognized that the successful application of small molecules in cancer therapy requires the identification of agents that, when combined, lead to synergistic tumor inhibition without significant systemic toxicity (Mayer,'07; Ramaswamy,'07). Therefore, the application of methods to quantitatively measure the nature of drug interaction endows a rational design of chemotherapy protocols (Chou,'11). However, such does not guarantee the successful applications. New approaches are needed to ensure that the chosen combination is in fact delivered to the target site.

### Nanotechnology-based platforms for drug delivery and diagnostics

The advent of nanotechnology brought the promise to revolutionize many fields in science by introducing the possibility to manipulate different materials at the nanoscale size rendering a variety of structures with different applications in areas such as cell-based therapies or cancer therapy and diagnosis. Nanocarriers represent one of the byproducts that emerged from nanotechnology research, holding the promise to revolutionize cancer treatment (Peer,'07). Numerous nanoparticles have been developed, including carbon nanotubes, polymeric carriers, dendrimers, metal particles (ex. gold nanoshells) and lipid-based particles (ex. liposomes) (Peer,'07). Of all mentioned particles, liposomes are currently one of the most advanced systems for drug delivery with several formulations approved for clinical use (Wang,'12).

Liposomes are vesicular organized structures, i.e. phospholipid membrane bilayers surrounding an inner aqueous core (Allen,'98). The success of these systems relies on their biocompatible and biodegradable composition, which further enables the encapsulation of hydrophilic agents in the inner aqueous core or hydrophobic drugs in the membrane bilayer, thus protecting them from degradation (Allen,'98; Wang,'12). Additionally, liposomes modify the pharmacokinetic and biodistribution profiles of the encapsulated drugs (Allen,'98). To exert their action at the tumor level, nanoparticles, including liposomes, take advantage of a phenomenon known as Enhanced Permeability and Retention effect (EPR). The specific tumor structure presents an extensive network of dysfunctional and leaky blood vessels, resulting from constantly activated angiogenesis, a process that conveys the formation of new blood vessels from the existing ones. The leaky vessel structure (with fenestrae up to 600 nm) combined with poor tumor lymphatic drainage originates the EPR effect, allowing the passive accumulation of nanoparticles at the tumor site (Peer,'07; Schroeder,'12; Wang,'12). Nonetheless, one can only take fully advantage of EPR effect if the nanoparticles, such as liposomes, present long blood circulation times.

The "first generation" of liposomal formulations presented a rapid blood clearance, along with accumulation into the mononuclear phagocyte system (MPS), following opsonization (Immordino,'06; Wang,'12). Long circulating liposomes emerged afterwards as a "second generation", by modulation of lipid composition and, specially, by the incorporation of poly-(ethyleneglycol) - "PEG" - onto the liposomal surface. PEG creates a hydrophilic cloud around the particle, thus minimizing the opsonization process and clearance by the MPS, rendering a technology known as Stealth® liposomes (Immordino,'06). The same strategy has been applied to other particles in order to increase their blood circulation times (Peer,'07). However, passive targeting does not fully address the issue of specific drug delivery to cancer cells due to randomness of the process, which may account for multidrug resistance (Peer,'07). In order to circumvent such limitation, one can actually further modify the nanoparticle surface with internalizing targeting moieties (Peer,'07; Wang,'12). Several targeting moieties have been used, including antibodies, Fab' and F(ab')₂ fragments, aptamers, small peptides or nanobodies, among others, which recognize overexpressed receptors at the cell surface and promote active internalization of the nanoparticles (Peer,'07). For example, Gao *et al.* used RGD peptide to promote targeted delivery of quantum dots for tumor fluorescence imaging (Gao,'10). Additionally, the same authors reported the utilization of recombinant Fab' antibody fragments against HER2 receptor for the delivery of PE38KDEL loaded liposomes to breast cancer cells (Gao,'09). A similar strategy was pursued by Simard *et al.* to intracellularly deliver arabinofuranosylcytosine to human myeloid leukemia cells using liposomes conjugated to anti-CD33 (Simard,'10). Despite increasing the drug accumulation into the target cell (*in vitro*), intracellular delivery does not necessarily guarantees increased bioavailability, as the drug (functionally active) has to escape from the endosomal compartment (Sapra,'03). Furthermore, the inclusion of PEGylated lipids onto the liposomal membrane also carries some disadvantages by restricting the interaction of the liposomes with the cell membrane, thus limiting cellular uptake and endosomal escape, a critical step for drugs that degrade at acidic conditions, like siRNAs (Gomes-da-Silva,'12).

Liposomal strategies have been rationally designed to take advantage of the progressive acidification of the endosomal environment upon internalization, giving rise of the so-called pH-sensitive liposomes. A common approach to produce pH-sensitive liposomes relies on the inclusion of phosphatidylethanolamine lipids, like dioleoylphosphatidylethanolamine (DOPE) combined with an amphiphilic stabilizing lipid. When isolated, DOPE adopts an inverted hexagonal phase (Hₗₗ) incompatible with membrane bilayer formation. The inverted hexagonal phase can be reverted by the inclusion of cholesteryl hemissuccinate (CHEMS), a cholesterol derivative with an acidic group, which presents a variable level of protonation according to the pH. The anionic form of CHEMS (at neutral to basic pH) reverts the hexagonal phase of DOPE to a lamellar phase (La) by increasing the hydration area at the lipid-water interface (Hafez,'00). As liposomes progress through the endocytic route, the increasing acidification of the medium protonates and neutralizes CHEMS, thus decreasing its stabilizing effect. Consequently, the system adopts the inverted hexagonal phase imposed by DOPE, leading to a disruption of the liposomal bilayer, fusion with the endosomal membrane, and liposomal content release into the cytosol (Simoes,'04). Upon modulating the DOPE/CHEMS ratio in the liposome bilayer, one can achieve a controlled/triggered intracellular release of the liposomal content.

### Specific Applications

The prior art teaches the combined use of doxorubicin and C6 ceramide. *Exogenous cell-permeable C6 ceramide sensitizes multiple cancer cell lines to Doxorubicin-induced apoptosis by promoting AMPK activation and mTORC1 inhibition. (http:doi: 10.1038*/*onc.2010.379).* This document demonstrates the therapeutic potential of the combination of doxorubicin and C6-ceramide against different types of cancer, focusing on the elucidation of the mechanism of action. This document, however, does not teach any strategy to translate the *in vitro* data to the *in vivo* setting. The different pharmacokinetic profiles of each drug would affect the therapeutic outcome, and the therapeutic value of any combination is not predictable from this document. The latter argument also applies to the manuscript *Pharmacodynamics and tumoricidal effect of adriamycin entrapped in ceramide sulphate-containing liposomes* (Biol. Pharm. Bull., 17(9) 1246-1250, 1994), where negatively charged ceramides have been incorporated in non-pH sensitive multilamellar liposomes. without any sort of targeting ligand. WO-A-2006/051549 discloses the use of a "single population liposome" for the treatment of cancer. Such a liposome has a lipid membrane comprising a lipid matrix, a pro-apoptotic C2-C8 ceramide, and a lipopolymer, and encapsulates in the intraliposomal aqueous phase an anticancer drug, preferably doxorubicin. There are also instances in the prior art of combining doxorubicin with non-cytotoxic ceramides. In the following manuscripts, the authors used glucosylceramide derivatives, either free, liposomal or co-encapsulating with doxorubicin, aiming at increasing the permeability of the cell membrane to amphiphilic compounds, as doxorubicin. Indeed, glucosylceramides are non-toxic sphingolipids. Therefore, these references are modulating drug delivery of the cytotoxic drug, but not delivering two cytotoxic drugs. [See: van Lummel, M., et al. (2011). "Enriching lipid nanovesicles with short-chain glucosylceramide improves doxorubicin delivery and efficacy in solid tumors." FASEB J 25(1): 280-289; Veldman, R. J., et al. (2005). "Coformulated N-octanoyl-glucosylceramide improves cellular delivery and cytotoxicity of liposomal doxorubicin." J Pharmacol Exp Ther 315(2): 704-710; Veldman, R. J., et al. (2004). "N-hexanoyl-sphingomyelin potentiates in vitro doxorubicin cytotoxicity by enhancing its cellular influx." Br J Cancer 90(4): 917-925.] WO-A-2009/142525 discloses targeted pH sensitive liposomes containing cytotoxic agents, such as doxorubicin, for use in the treatment of cancer. The prior art also teaches the importance of cancer stem cells in disease. The prior art also discusses that cancer stem cells share features of normal stem cells, however definitive markers for cancer stem cells are still under debate. Nonetheless, the prior art teaches targeting cancer stem cells with nanoparticle enabled therapies. The reference *Targeting Cancer Stem Cells with Nanoparticle-Enabled Therapies* presents examples of particles used for drug delivery, including an example of particles targeted by CD44 antibody, a marker used for the identification of cancer stem cells. (http://dx.doi.org/10.4172/2155-9929.S8-003)

Nucleolin has been shown to be present on certain cancer cells. It has been used as a target for the treatment of cancer using pH sensitive liposomes that include a targeting ligand that binds nucleolin. [See U.S. 8529944] Nucleolin also has been used to track cancer cells. The reference A Nucleolin-Targeted Multimodal Nanoparticle Imaging Probe for Tracking Cancer Cells Using an Aptamer (doi: 10.2967/jnumed.109.069880) teaches a non-lipid based multimodal imaging nanoparticle (cobalt-ferrite based), which targets cell-surface nucleolin using the AS1411 aptamer. Nucleolin has not been demonstrated, however, to be present on the surface of cancer stem cells.

Besides the presence of nucleolin on certain cancer cells, it has been described to have a role in mouse embryonic stem cells (Yang, Shi et al. 2011). This paper, however, does not discuss whether nucleolin is present on the surface of these stem cells. Nor is the same predictable, as nucleolin suffers several post-translational modifications (Carpentier, Morelle et al. 2005) that would affect whether the molecule would be expressed on the cell surface. In addition, these post-translational modifications differ in normal and disease conditions as cancer (Karve and Cheema 2011).

Although cancer stem cells and normal stem cells may share phenotypic or genetic features, it has not been demonstrated that *surface* nucleolin is present on both types of cells. Therefore, it was unknown and unpredictable whether a molecule that binds nucleolin, such as F3 peptide, could target cancer stem cells.

There is one published patent application, implying that nucleolin is present on the surface of cancer stem cells, but there is no data in the application suggesting that nucleolin is present on the surface of cancer stem cells. (See WO2009088837A2) However, this published application does not suggest exploiting nucleolin for intracellular drug delivery.

### SUMMARY OF THE INVENTION

The invention is defined by the claims. Any subject-matter falling outside the scope of the claims is provided for information purposes only. Any references in the description to methods of treatment refer to the compounds, pharmaceutical compositions and medicaments of the present invention for use in a method for treatment of the human or animal body by therapy. The present disclosure comprises a delivery system involving a targeting moiety and a nanostructure with a particle size lower than 300 nm, carrying an active agent or a combination of active agents, exhibiting a synergistic or non-synergistic activity, which mediates efficient and specific cellular delivery to stem cells (and/or tumor cells).

The present disclosure allows: i) the delivery of active agents, alone or in combination, aiming at modulation of stem cells (SC) and development of novel cell therapies ii) diagnosis, based on detection of SC that specifically interact with the delivery system; iii) delivery of active agents, alone or in combination, to induce death of cancer stem cells, aiming at cancer therapy; iv) delivery of a combination of diagnostic agents and active agents to stem cells and/or tumor cells, thus allowing theranostics.

The present invention provides a delivery system capable of encapsulate and maintain overtime a synergistic or non-synergistic drug combination. The system is armed on the surface with an internalizing targeting moiety, which enables specific, receptor-mediated endocytosis into stem cells, including cancer stem cells and/or other tumor cells as well, providing a major improvement in tumor therapy. It enables the delivery of a synergistic drug combination preferentially and/or specifically to cancer stem cells, a drug resistant cell population responsible for sustaining tumor growth. The targeted delivery of synergistic drug combination enables a decrease of toxic side effects. Furthermore, in a non-claimed aspect of the disclosure, the proposed system can bind to stem cells enabling the specific delivery of active agents, either alone or in combination, aiming at stem cell modulation and novel cell-based therapies. Additionally, the specific interaction of the system with stem cells enables their detection representing a promising feature for diagnosis purposes.

Therefore, theranostics is within reach of such system, enabled by the delivery of a combination of diagnostic and active agents to cancer stem cells and tumor cells.

The versatility of this platform enables the modification of the nanosystem, in terms of its composition, enclosed agent or the ligand, according to its purpose (either treatment or diagnosis) and/or disease.

It was discovered, surprisingly, that nucleolin is expressed on the surface of cancer stem cells and that this can be exploited to target cancer stem cells. It was discovered, surprisingly, that a pH sensitive liposomes formed in part from cytotoxic ceramide analogs, still maintain sufficient integrity in vitro and, we predict, in vivo to permit their therapeutic use. The liposomes are stable in vitro and in vivo, maintain their payload and releasing their payload at the desired pH. It was discovered, surprisingly, that cytotoxic ceramide analogs are stable in the endosome pathway, making them available as a cytotoxic agent when the liposome disintegrates at the desired pH.

In addition, it was discovered, surprisingly, that the most desirable molar concentration of drug combinations in vitro turned out to be less desirable. In particular, DXR:C6-ceramide in a 1:2 molar ratio in vitro was synergistic whereas DXR:C6-Ceramide in a 1:1 molar ratio in vitro was antagonist/mildly additive. However, when placed into targeted, pH-sensitive liposomes, DXR:C6-Ceramide in a 1:1 molar ratio was even more active than the synergistic DXR:C6-ceramide 1:2 molar ratio.

According to one aspect of the invention, a ligand-targeted delivery system as defined in the claims is provided. The system includes a targeting ligand that binds a tumor cell, including a cancer cell, linked to a support carrying an agent, wherein said support is a pH sensitive liposome having a pro-apoptotic lipid, which is a cytotoxic ceramide analog as defined in claim 1, encapsulated, entrapped or intercalated in the support, wherein the agent is doxorubicin encapsulated in the support, wherein the molar ratio of cytotoxic ceramide analog to doxorubicin is between 3:1 and 1:2, and wherein said liposome is capable of the pH dependent intracellular release of said pro-apoptotic lipid and said agent. The cytotoxic ceramide analog is selected from the group consisting of N-acetoyl-D-erythro-sphingosine, N-butyroyl-D-erythro-sphingosine, N-hexanoyl-D-erythro-sphingosine, N-octanoyl-D-erythro-sphingosine, N-decanoyl-D-erythro-sphingosine, N-lauroyl-D-erythro-sphingosine, N-myristoyl-D-erythro-sphingosine, N-palmitoyl-D-erythro-sphingosine. In embodiments, the cytotoxic ceramide analog can be N-hexanoyl-D-erythro-sphingosine (C6-Ceramide), N-octanoyl-D-erythro-sphingosine and/or N-palmitoyl-D-erythro-sphingosine. In embodiments, the cytotoxic ceramide analog is C6-Ceramide and the molar ratio of C6-ceramide to doxorubicin can in some embodiments be less than 2:1, be between 1.5:1 and 1:1.5 or be about 1:1. In embodiments, the targeting ligand can bind a cancer stem cell.

The targeting ligand can be any targeting ligand that selectively binds to the surface of a tumor cell. This includes peptides, antibodies, nanobodies, receptors, small molecules, aptamers and the like. These ligands can bind molecules on tumor cells that permit selective binding to the tumor cell specific, such as those listed in the background of the invention and below. In embodiments, the targeting ligand recognizes and binds preferentially a cancer stem cell. In embodiments, the targeting ligand binds nucleolin . In embodiments, the targeting ligand is a peptide comprising the amino acid sequence of SEQ ID NO:1.

The targeting ligand can be covalently or non-covalently attached to the support. In embodiments, the targeting ligand is linked to the support by covalent attachment to a spacer positioned between the targeting ligand and the support, such that the interaction of the ligand with the target is not hindered. In embodiments, the targeting ligand is part of a molecule that comprises the targeting ligand, a spacer and a lipid which forms part of the liposome.

The support is a pH sensitive liposome. The liposome can be formed of any of the materials conventionally used to form liposomes, many of which are listed below.

In embodiments, the liposome can include 1,2-dioleoyl-sn-glycero-3-phosphoethanolamine; 3β-hydroxy-5-cholestene-3-hemisuccinate; 1,2-distearoyl-sn-glycero-3-phosphocholine; cholesterol; 1,2-distearoyl-sn-glycero-3-phosphoethanolamine-N-[methoxy(polyethylene glycol)-2000]; and a cytotoxic ceramide analog. In embodiments, the liposome can include 1,2-dioleoyl-sn-glycero-3-phosphoethanolamine; 3β-hydroxy-5-cholestene-3-hemisuccinate; 1,2-distearoyl-sn-glycero-3-phosphocholine; cholesterol; 1,2-distearoyl-sn-glycero-3-phosphoethanolamine-N-[methoxy(polyethylene glycol)-2000]; and cytotoxic ceramide analog in a mol% ranging from 37 mol% to 5 mol%, 19 mol% to 2 mol%, 9 mol% to 31 mol%, 7 mol% to 4 mol% and from 19 mol% to 28 mol%, respectively. In one embodiment it is a mol% of approximately 37:19:9:9:7:19.

In embodiments, the pH sensitive liposome permits the intracellular triggered release of the agent as a function of the pH value of the target microenvironment and wherein the agent is released through the support destabilization in acidic environment. In embodiments, the acidic environment comprises the endosome compartment of cells.

In embodiments, the delivery system is suitable for intravenous administration.

According to another aspect of the invention, a ligand-targeted delivery system as disclosed in the claims is provided. The system includes a targeting ligand that binds a tumor cell, including a cancer cell linked to a support for carrying a cytotoxic agent encapsulated, entrapped or intercalated in the support, wherein said support is a pH sensitive liposome having a cytotoxic ceramide analog intercalated in the support, and wherein said support is capable of the pH dependent intracellular release of said cytotoxic ceramide analog and said agent, as defined in the claims. In embodiments, the nanosystem is a pH-sensitive liposome. In embodiments, the nanosystem is a liposome made of one or two or three or more of: fully hydrogenated soy phosphatidylcholine, methoxy-polyethylene glycol phosphatidylethanolamine, maleimide-polyethylene glycol phosphatidylethanolamine, N-methylpalmitoyloleoylphosphatidylcholine, phosphatidylserine, phosphatidylcholine, palmitoyloleoylphosphatidylcholine, dipalmitoylphosphatidylcholine, sphingomyelin, distearoylphosphatidylcholine, diphytanoylphosphatidylcholine, N-acetoyl-D-erythro-sphingosine, N-butyroyl-D-erythro-sphingosine, N-hexanoyl-D-erythro-sphingosine, N-octanoyl-D-erythro-sphingosine, N-decanoyl-D-erythro-sphingosine, N-lauroyl-D-erythro-sphingosine, N-myristoyl-D-erythro-sphingosine, N-palmitoyl-D-erythro-sphingosine, phosphatidylglycerol, dioleoylphosphatidylethanolamine, 1,2-dioleoyl-3-trimethylammonium-propane, N-palmitoyl-sphingosine-1-(succinyl[methoxy(polyethylene glycol)]), L-α-Phosphatidylethanolamine-N-(lissamine rhodamine B sulfonyl), cholesteryl hemisuccinate, or cholesterol. In embodiments, the nanosystem is a liposome including, but not limited to 1,2-dioleoyl-sn-glycero-3-phosphoethanolamine; 3β-hydroxy-5-cholestene-3-hemisuccinate; 1,2-distearoyl-sn-glycero-3-phosphocholine; cholesterol; 1,2-distearoyl-sn-glycero-3-phosphoethanolamine-N-[methoxy(polyethylene glycol)-2000]; and cytotoxic ceramide analog. In embodiments, the support is a liposome comprising 1,2-dioleoyl-sn-glycero-3-phosphoethanolamine; 3β-hydroxy-5-cholestene-3-hemisuccinate; 1,2-distearoyl-sn-glycero-3-phosphocholine; cholesterol; 1,2-distearoyl-sn-glycero-3-phosphoethanolamine-N-[methoxy(polyethylene glycol)-2000]; and cytotoxic ceramide analog in a mol% ranging from 37 mol% to 5 mol%, 19 mol% to 2 mol%, 9 mol% to 31 mol%, 7 mol% to 4 mol% and from 19 mol% to 28 mol%, respectively. In one embodiment it is a mol% of approximately 37:19:9:9:7:19. The cytotoxic ceramide analog is as defined in the claims. In embodiments, the cytotoxic ceramide analog is N-hexanoyl-D-erythro-sphingosine, N-octanoyl-D-erythro-sphingosine and/or N-palmitoyl-D-erythro-sphingosine.

In embodiments, the pH sensitive liposome permits the intracellular triggered release of the agent as a function of the pH value of the target microenvironment and wherein the agent is released through the support destabilization in acidic environment. In embodiments, the acidic environment comprises the endosome compartment of cells.

The targeting ligand can be any targeting ligand that specifically binds to the surface of a tumor cell. This includes peptides, antibodies, nanobodies, receptors, small molecules, aptamers and the like. These ligands can bind molecules on tumor cells that permit selective binding to the tumor cell, such as, but not limited to CD19, sigma receptor, HER2 (human epidermal growth factor receptor 2), epidermal growth factor receptor, Nucleolin, PSMA (prostate specific membrane antigen), SSEA1 (stage-specific embryonic antigen 1), SSEA3/4 (stage-specific embryonic antigen 3/4), TRA-1-60 (tumor-related antigen 1-60), TRA-1-81 (tumor-related antigen 1-81), GCTM2, GCT343, CD9, Thy1 (a.k.a. CD90), HLA1 (human leucocyte antigen 1), CD24, CD44, CD133, EpCAM (epithelial cell adhesion molecule), CD34, CD38, epithelial membrane protein, and vascular endothelial growth factor receptor, pituitary adenylate cyclase-activating peptide receptors, lymphatic vessel endothelial hyaluronan receptors, prohibitin, thrombin receptor protease-activated receptor type 1, Notch, integrin receptors, lectin receptor, lactoferrin receptor, annexin 1, platelet-derived growth factor receptors, ephrin receptors as, but not limited to, the ephrinA4 receptor, C-kit, glycoprotein 60, aminopeptidase A, aminopeptidase N, CD13, endosialin, plectin-1, p32/gC1q receptor, fibronectin ED-B, fibrin-fibronectin complexes, interleukin 11 receptor alpha, protease -cleaved collagen IV, hyaluronic binding protein, NG2 proteoglycan, prohibitin, heat shock protein 90, neuropilin 1, neuropilin 2, matrix metalloproteinase 2, matrix metalloproteinase 9. In embodiments, the targeting ligand recognizes and binds preferentially a cancer stem cell. In embodiments, the targeting ligand binds nucleolin. In embodiments, the targeting ligand is a peptide comprising the amino acid sequence of SEQ ID NO:1.

The targeting ligand can be covalently or non-covalently attached to the support. In embodiments, the targeting ligand is linked to the support by covalent attachment to a spacer positioned between the targeting ligand and the support, such that the interaction of the ligand with the target is not hindered. In embodiments, the targeting ligand is part of a molecule that comprises the targeting ligand, a spacer and a lipid which forms part of the liposome.

In a non-claimed aspect of the disclosure, the cytotoxic agent is one or more selected from the group consisting of alkylating drugs; cytotoxic antibiotics; antimetabolites; vinca alkaloids; amsacrine; altetarmine; crisantaspase; dacarbazine; temozolomide; hydroxycarbamide (hydroxyurea); pentostatin; platinum compounds; porfimer sodium; procarbazine; razoxane; taxanes; topoisomerase I inhibitors; trastuzumab; tretinoin; SN-38; ET-743; TLK 286; anti-inflammatory agents; antiangiogenic agents or angiolytic agents; ABT-627; Bay 12-9566; Benefin; Bevacizumab; BMS-275291; cartilage-derived inhibitor(CDI); CAI; CD59 complement fragment; CEP-7055; Col 3; Combretastatin A-4; Endostatin (collagenXVIII fragment); Fibronectin fragment; Gro-beta; Halofuginone; Heparinases; Heparin hexasaccharide fragment; HMV833; Human chorionic gonadotropin (hCG); IM-862; Interferon alpha/beta/gamma; Interferon inducible protein (IP-10); Interleukin-12; Kringle 5 (plasminogen fragment); Marimastat; Metalloproteinase inhibitors (TIMPs); 2-Methoxyestradiol; MMI 270 (CGS 27023A); MoAbIMC-1C11; Neovastat; NM-3; Panzem; PI-88; Placental ribonuclease inhibitor; Plasminogen activator inhibitor; Platelet factor-4 (PF4); Prinomastat; Prolactin16kD fragment; Proliferin-related protein (PRP); PTK 787/ZK 222594; Retinoids; Solimastat; Squalamine; SS 3304; SU 5416; 5U6668; SU11248; Tetrahydrocortisol-S; tetrathiomolybdate; thalidomide; Thrombospondin-1(TSP-1); TNP-470; Transforming growth factor-beta (TGF-b); Vasculostatin; Vasostatin (calreticulin fragment); ZD6126; ZD 6474; farnesyl transferase inhibitors (FTI); bisphosphonates; and porphyrins. The cytotoxic agent also may be one or more quinolones (including, but not limited to, ciprofloxacin and trovafloxacin); and/or tyrosine kinase inhibitors (TKI), (including, but not limited to, imatinib, dasatinib, nilotinib, bosutinib, lapatinib, gefitinib, erlotinib, vandetanib, vemurafenib, crizotinib, sorafenib, sunitinib, pazopanib, regorafenib, cabozantinib).

According to another aspect of the disclosure, a method for killing a cancer stem cell is provided. The method involves contacting a cancer stem cell with any one of the nanosystems described above, wherein the agent is a cytotoxic drug. The nanosystems are administered in amounts effective to kill the cancer stem cell. In embodiments, the contacting occurs as a result of administering the nanosystem to a subject, wherein the subject is a mammal, including, but not limited to, a human. In some embodiments, the cancer stem cell is a human stem cell.

According to another non-claimed aspect of the disclosure, a method for delivering a drug to a stem cell that is not a cancer stem cell is provided. The method involves contacting a stem cell with any one of the nanosystems described above, wherein the agent is not a cytotoxic drug. In embodiments, the agent can be any one of the agents described herein for modifying the activity or function of a stem cell. In embodiments, the agent comprises a nucleic acid encoding a protein. The nanosystems are administered in amounts effective to modifying the activity or function of the stem cell. In embodiments, the contacting occurs as a result of administering the nanosystem to a subject, wherein the subject is a mammal, including, but not limited to, a human. In some embodiments, the stem cell is a human stem cell.

According to another non-claimed aspect of the disclosure, a method for identifying a stem cell is provided. The method involves contacting a plurality of cells only some of which are stem cells with any one of the nanosystems described above, wherein the agent is a label. The label can be any label conventionally used in identifying cells, such as a fluorescent labeled molecule, a dye, a radioactive labeled molecule, and the like. In embodiments, the agent can be any one of the labels described herein. The nanosystems are administered in amounts effective to permit detection of the stem cell. In embodiments, the contacting occurs as a result of administering the nanosystem to a subject, wherein the subject is a mammal, including, but not limited to, a human. In some embodiments, the stem cell is a human stem cell. In embodiments involving administration, the nanosystems are prepared typically as sterile, injectable formulations in pharmaceutically acceptable carriers. Other preparations may be used, including but not limited to, as topical and inhalable preparations. The nanosystems are administered for treatment in effective amounts. An effective amount means amounts in one or more dosages which have the intended therapeutic of diagnostic effect. In general, it is an amount sufficient to ameliorate one or more symptoms or manifestations of disease.

According to another non-claimed aspect of the disclosure, a method to deliver a drug to human stem cells that are not cancer stem cells is provided. The method involves administering to a subject a nanotechnology-based platform comprising at least one ligand ornamenting a nanosystem carrying a drug, suitable for systemic administration into mammals, the ligand binding a target overexpressed on the surface of stem cells, wherein the target is nucleolin, and wherein the nanosystem is a cell, quantum dot, magnetic particle, gold nanoparticle, nanoshell, carbon nanotube, lipid-based particle, virus, polymeric particle such as microcapsule, biodegradable microdevice, agarose, or gelatin. In embodiments, the drug is enclosed within the nanosystem. In embodiments, the stem cell is not a cancer stem cell and the drug is, but not limited to, VPA, NaB, BIX-01294, parnate, RG108, PD0325901, PD98059, A38-01, SB431542, CHIR99021, Kenpaullone, BayK 8644, Vitamine C, Thiazovivin, Y-27632, apigenin, E-616452, EI-275, Forskolin, PS48, LY294002, stauprimide, IDE1, indolactam V, StemRegenin1, AMD3100, or diprotin A.

According to another non-claimed aspect of the disclosure, a method for imaging stem cells in a subject is provided. The method involves administering to a subject a nanotechnology-based platform comprising at least one ligand ornamenting a nanosystem carrying a label, suitable for systemic administration into mammals, the ligand binding a target overexpressed on the surface of stem cells, wherein the target is nucleolin, and wherein the nanosystem is a cell, quantum dot, magnetic particle, gold nanoparticle, nanoshell, carbon nanotube, lipid-based particle, virus, polymeric particle such as microcapsule, biodegradable microdevice, agarose, or gelatin. In embodiments, the label is enclosed within the nanosystem. In embodiments, the stem cells are cancer stem cells. In embodiments, the label is a radioisotope or a fluorescent molecule.

In embodiments of the foregoing methods, the nanosystem can be a lipid-based particle. In embodiments, the nanosystem is a liposome. In embodiments, the nanosystem is a pH-sensitive liposome. In embodiments, nanosystem is a liposome made of one or two or three or more of: fully hydrogenated soy phosphatidylcholine, methoxy-polyethylene glycol phosphatidylethanolamine, maleimide-polyethylene glycol phosphatidylethanolamine, N-methylpalmitoyloleoylphosphatidylcholine, phosphatidylserine, phosphatidylcholine, palmitoyloleoylphosphatidylcholine, dipalmitoylphosphatidylcholine, sphingomyelin, distearoylphosphatidylcholine, diphytanoylphosphatidylcholine, N-acetoyl-D-erythro-sphingosine, N-butyroyl-D-erythro-sphingosine, N-hexanoyl-D-erythro-sphingosine, N-octanoyl-D-erythro-sphingosine, N-decanoyl-D-erythro-sphingosine, N-lauroyl-D-erythro-sphingosine, N-myristoyl-D-erythro-sphingosine, N-palmitoyl-D-erythro-sphingosine, phosphatidylglycerol, dioleoylphosphatidylethanolamine, 1,2-dioleoyl-3-trimethylammonium-propane, N-palmitoyl-sphingosine-1-(succinyl[methoxy(polyethylene glycol)]), L-α-Phosphatidylethanolamine-N-(lissamine rhodamine B sulfonyl), cholesteryl hemisuccinate, or cholesterol. These and other aspects of the invention are described in greater detail below.

### BRIEF DESCRIPTION OF THE DRAWINGS

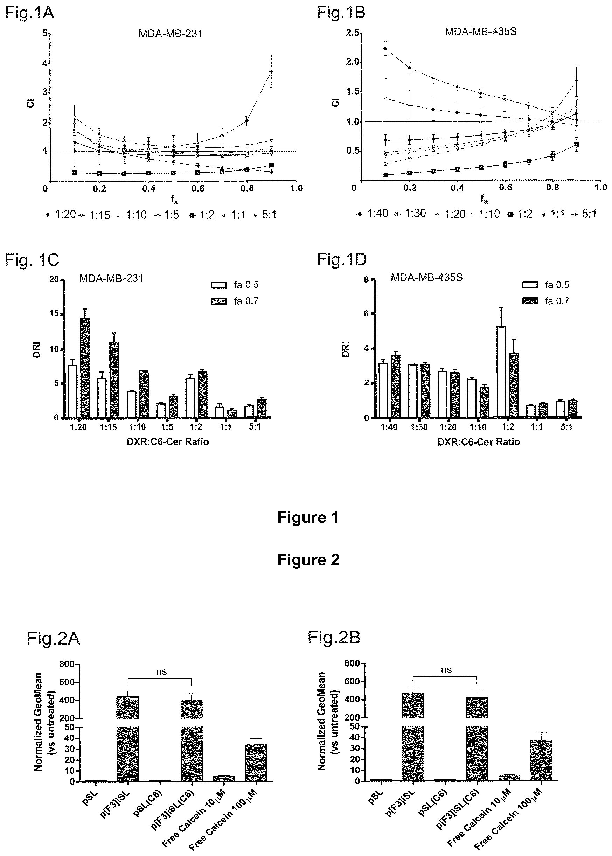

Figures 1A-1D are graphic representations of in vitro screening for synergy of different doxorubicin (DXR):C6-ceramide (C6-Cer) ratios. Fig. 1(A) and Fig. 1(B) represent the combination index versus fraction affected (fa) at different DXR:C6-Cer drug ratios for MDA-MB-231 and MDA-MB-435S cell lines, respectively. Fig 1(C) and Fig. 1(D) represent the Dose Reduction Index (DRI) of doxorubicin achieved with different DXR:C6-Cer ratios at IC₅₀ (fa 0.5) and IC₇₀ (fa 0.7) for doxorubicin in MDA-MB-231 and MDA-MB-435S cell lines, respectively. Combination Index (CI) as a measure of interaction between drugs was used, where Cl values <1, >1 or ≈1 indicate synergy, antagonism and additivity, respectively. Mean Combination Index ± SEM was calculated from data of 3-6 independent experiments performed in triplicate. Dose reduction index (DRI) as a measure of a dose fold-reduction is obtained by the ratio of each drug alone *versus* the drug in a combination, both producing the similar effect "fa". Mean DRI ± SEM was calculated from data of 3-6 experiments, each performed in triplicate.
Figures 2A and 2B are graphs showing the effect of membrane-incorporated ceramide on liposomal pH-sensitivity. Two hundred thousand MDA-MB-435S and MDA-MB-231 breast cancer cells were incubated with either 50 µM of liposomes encapsulating calcein or free calcein at the indicated concentrations for 1 h at 37°C and immediately analyzed through a FACScalibur flow cytometer. Fig. 2A and Fig. 2B represent the calcein geometric mean fluorescence normalized against the respective signal of the untreated control for MDA-MB-435S and MDA-MB-231 cell lines, respectively. Data represents the mean ± SEM of 3 experiments (One-Way ANOVA p<0.001; ns p>0.05 Tukey's Multiple Comparison Test). pSL-non-targeted liposomes encapsulating calcein; p[F3]iSL - F3 peptide-targeted liposomes encapsulating calcein (without ceramide). pSL(C6) - non-targeted liposomes co-encapsulating ceramide and calcein; p[F3]iSL(C6) - F3 peptide targeted liposomes co-encapsulating ceramide and calcein.

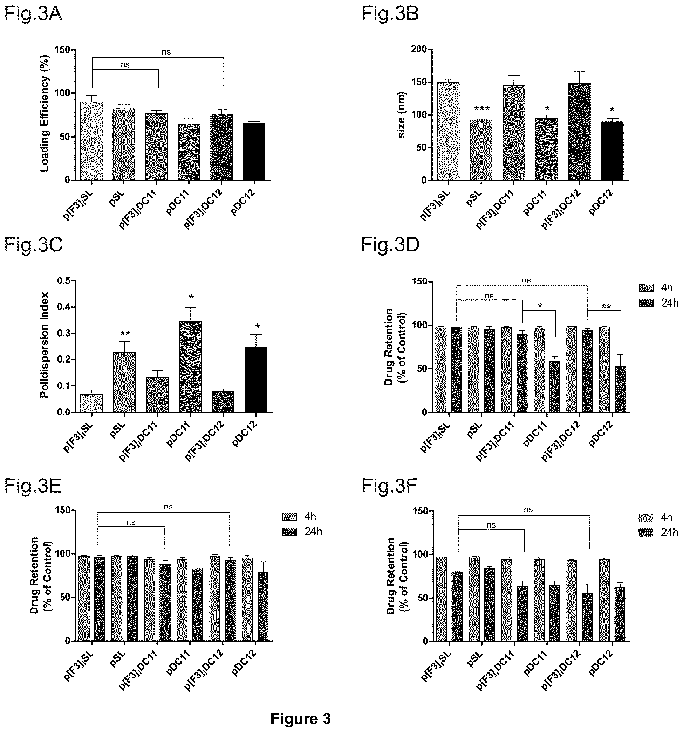

Figure 3A-F are graphs characterizing liposomes encapsulating different Doxorubicin (DXR):C6-ceramide (C6-Cer) ratios. Effect of Ceramide on 3A Loading efficiency (1-Way ANOVA p>0.05, ns p>0.05 Tukey's multicomparison test), 3B size (***p<0.0001, *p<0.05, t test of targeted liposomes vs non-targeted), and 3C polydispersion index (**p<0.01, *p<0.05; t test of targeted vs non-targeted liposomes) of F3-targeted and non-targeted liposomes loaded at the indicated DXR:C6-Cer ratios. 3D, 3E and 3F represent the drug retention capacity of each liposomal formulation when incubated at 37°C in HBS pH 7.4, RPMI 1640 supplemented with 10% FBS or in 90% non-inactivated serum, respectively (**p<0.01, *p<0.05, ns p>0.05 by Tukey's multicomparison). Data represent mean ± SEM from 3 to 8 independent experiments. p[F3]iSL and pSL - targeted and non-targeted liposomes without ceramide, respectively. p[F3]iDC11 and pDC11 - targeted and non-targeted liposomes encapsulating the DXR:C6-Cer 1:1 molar ratio, respectively. p[F3]iDC12 and pDC12 - targeted and non-targeted liposomes encapsulating the DXR:C6-Cer 1:2 molar ratio, respectively.

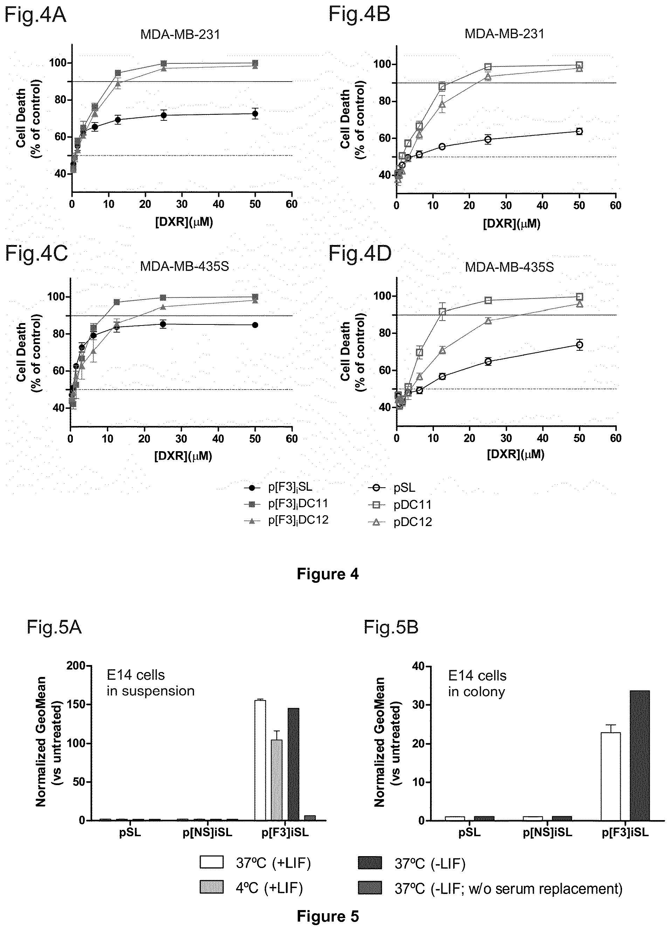

Figures 4A-D are graphs showing the cytotoxicity of combinations of doxorubicin and C6-ceramide encapsulated in different liposomal formulations. Cells were incubated for 24 h with the different liposomal drugs at DXR serially diluted concentrations, and the experiment was further prolonged for 72 h after which cell death was assessed. Figures represent the dose-response curves for 24 h of incubation of F3 peptide-targeted or non-targeted liposomes for the MDA-MB-231 breast cancer cell line (4A and 4B, respectively) or for the MDA-MB-435 breast cancer cell line (4C and 4D, respectively), either encapsulating the DXR:C6-ceramide selected ratios or doxorubicin only. The data points represent the mean ± SEM of 3 independent experiments for each concentration tested. Doted-line: 50% of cell death. Line: represents the 90% of cell death. p[F3]iSL and pSL - targeted and non-targeted liposomes without ceramide, respectively. p[F3]iDC11 and pDC11 - targeted and non-targeted liposomes encapsulating the DXR:C6-cer 1:1 ratio, respectively. p[F3]iDC12 and pDC12 - targeted and non-targeted liposomes encapsulating the DXR:C6-cer 1:2 ratio, respectively.
Figures 5A and B are graphs showing cellular association of F3-targeted liposomes with mESC. E14 mouse embryonic stem cells were incubated with 0.4 mM total lipid of pSL liposomes incorporating 1 mol% of Rhodamine-PE, for 1 h at 4°C or 37°C, and subsequently, analyzed by flow cytometry. 5A and 5B represent the geometric mean of rhodamine fluorescence for each system normalized against the respective signal of the untreated for E14 cells in suspension and in colony, respectively. Non-viable cells were excluded from the analysis using 7-AAD. pSL - non-targeted liposomes; p[NS]iSL - targeted liposomes by a non-specific peptide; p[F3]iSL - F3 peptide-targeted liposomes.

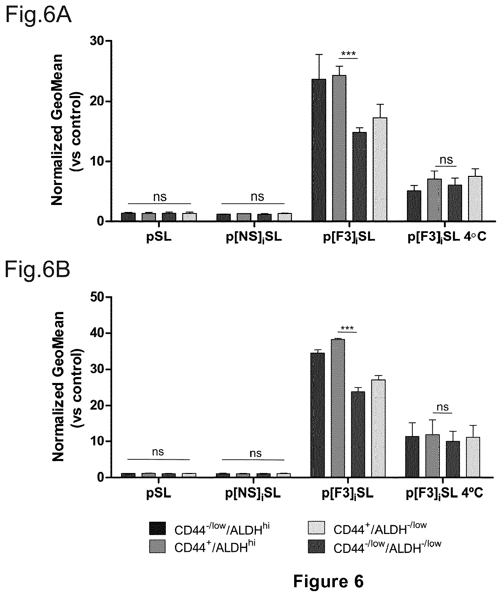

Figures 6A and B are graphs showing the cellular association of F3-targeted liposomes with cancer stem cells from breast cancer cell lines. Half a million MDA-MB-231 or MDA-MB-435S cells were incubated with 0.4 mM of Rhod-labelled liposomes for 1 h at 37°C or 4°C, and subsequently stained with anti-CD44-PECy5 antibody and with ALDH activity detection kit, ALDEFLUOR, and immediately analyzed through a FACScalibur flow cytometry system. 6A and 6B represent the rhodamine geometric mean fluorescence of each population normalized against the respective signal of the untreated control for MDA-MB-231 and MDA-MB-435S cell line, respectively (light-blue: cancer stem cells; orange: non-cancer stem cells). Data represent mean ± SEM of 3 independent experiments (2-Way ANOVA p<0.0001 for formulation variable; ns p>0.05 and ***p<0.001 Bonferroni's post test). pSL - non-targeted liposomes; p[NS]iSL - targeted liposomes by non-specific peptide; p[F3]iSL - F3 peptide-targeted liposomes.

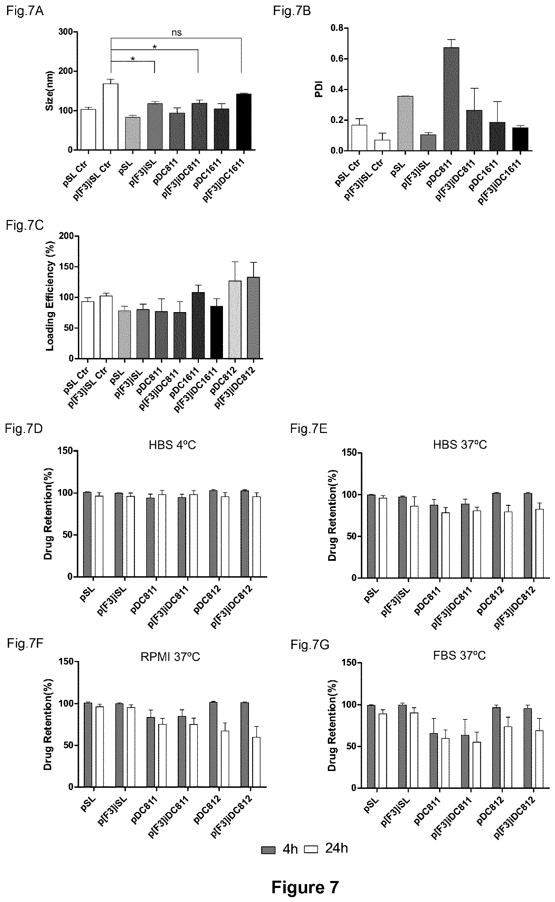

Figures 7A-7D are graphs characterizing liposomes prepared by an ethanol injection method and encapsulating Doxorubicin (DXR) and either C8-ceramide (C8-Cer) or C16-ceramide (C16-Cer). Effect of ethanol injection method on (7A) size [1-Way ANOVA p=0.0004; * p<0.05, ns p>0.05 Tukey's multicomparison test, (7B) polydispersion index (1-Way ANOVA p=0,0013), and (7C) loading efficiency (1-Way ANOVA p=0,181) of F3-targeted and non-targeted liposomes loaded at indicated DXR:C8-Cer and DXR:C16-Cer ratios compared to lipid film hydration method. (7D-7G) represent the drug retention capacity of each liposomal formulation when incubated at 4°C (2-Way ANOVA p>0.05 for both variables) or 37°C in HBS pH 7.4 (2-Way ANOVA, formulation: p=0.15, time: p=0.0011), RPMI 1640 supplemented with 10% FBS (2-Way ANOVA, formulation: p=0.013, time: p=0.0002) or in 90% non-inactivated serum (2-Way ANOVA, formulation: p=0.0075, time: p=0.0343), respectively. Data represent mean ± SEM from 2 to 7 independent experiments. p[F3]ᵢSL Ctr and pSL Ctr - targeted and non-targeted liposomes prepared by lipid film hydration method without ceramide, respectively. p[F3]ᵢSL and pSL - targeted and non-targeted liposomes without ceramide, respectively. p[F3]ᵢDC811 and pDC811 - targeted and non-targeted liposomes encapsulating the DXR:C8-Cer 1:1 molar ratio, respectively. p[F3]ᵢDC1611 and pDC1611 - targeted and non-targeted liposomes encapsulating the DXR:C16-Cer 1:1 molar ratio, respectively. p[F3]ᵢDC812 and pDC812 - targeted and non-targeted liposomes encapsulating the DXR:C8-Cer 1:2 molar ratio, respectively.

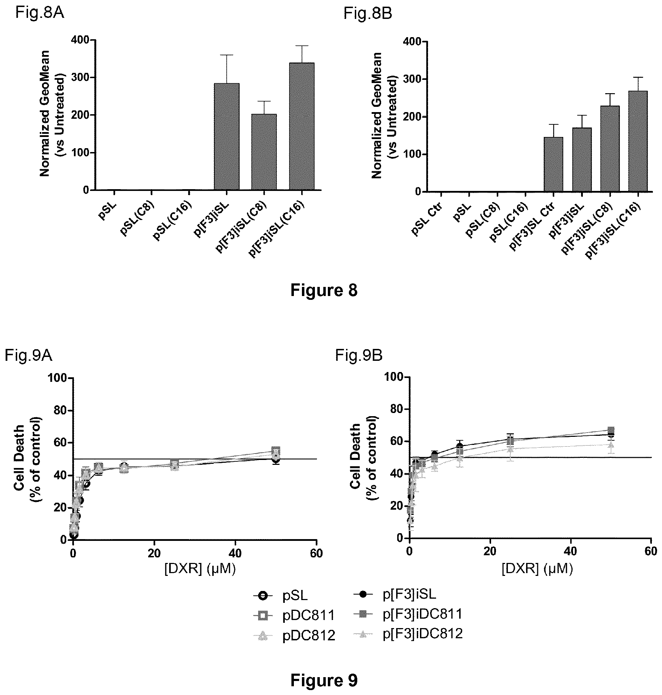

Figures 8A and 8B are graphs showing the effect of membrane-incorporated ceramide on liposomal pH-sensitivity of liposomes prepared by ethanol injection method. Two hundred thousand MDA-MB-435S and MDA-MB-231 breast cancer cells were incubated with 50 µM of liposomes encapsulating calcein or for 1 h at 37°C and immediately analyzed through a FACScalibur flow cytometer. 8A and 8B represents the calcein geometric mean fluorescence normalized against the respective signal of the untreated control for MDA-MB-231 and MDA-MB-435S cell lines, respectively. Data represents the mean ± SEM of 2-3 experiments (1-Way ANOVA p<0.0001). pSLCtr - non-targeted liposomes prepared by lipid film hydration method encapsulating calcein; p[F3]ᵢSL Ctr - F3 peptide-targeted liposomes prepared by lipid film hydration method encapsulating calcein (without ceramide).pSL - non-targeted liposomes encapsulating calcein; p[F3]ᵢSL - F3 peptide-targeted liposomes encapsulating calcein (without ceramide). pSL(C8) - non-targeted liposomes co-encapsulating C8-ceramide and calcein; p[F3]iSL(C8) - F3 peptide targeted liposomes co-encapsulating C8-ceramide and calcein. pSL(C16) - non-targeted liposomes co-encapsulating C16-ceramide and calcein; p[F3]iSL(C16) - F3 peptide targeted liposomes co-encapsulating C16-ceramide and calcein.
Figures 9A and 9B are graphs showing the cytotoxicity of combinations of doxorubicin and C8-ceramide encapsulated in different liposomal formulations prepared by ethanol injection method. Cells were incubated for 4 h with the different liposomal drugs at DXR serially diluted concentrations, and the experiment was further prolonged for 92 h after which cell death was assessed. Figures represent the dose-response curves for 4 h of incubation of non-targeted liposomes or F3 peptide-targeted for the MDA-MB-231 breast cancer cell line (9A and 9B, respectively) either encapsulating the DXR:C8-ceramide selected ratios or doxorubicin only. The data points represent the mean ± SEM of 3-5 independent experiments for each concentration tested. Line: 50% of cell death. p[F3]ᵢSL and pSL - targeted and non-targeted liposomes without ceramide, respectively. p[F3]ᵢDC811 and pDC811 - targeted and non-targeted liposomes encapsulating the DXR:C8-cer 1:1 ratio, respectively. p[F3]ᵢDC812 and pDC812 - targeted and non-targeted liposomes encapsulating the DXR:C8-cer 1:2 ratio, respectively.

### DETAILED DESCRIPTION OF THE INVENTION

The present disclosure in one aspect provides a nanotechnological platform (system) able to preferentially deliver, at the intracellular level, an agent or a combination of agents, either synergistic or not synergistic, to a specific subset of cells within a tumor (either, but not limited to, cancer cells, endothelial cell and/or cancer stem cells), achieved by coupling or inserting an internalizing targeting moiety (ligand) at the surface of the nanosystem. Additionally, the provided system allows specific delivery of an agent or a combination of agents, either synergistic or not, to stem cells, including but not restricted to embryonic stem cells of mouse (mESC) or human (hESC) origin, epiblast stem cells (EpiSC) or induced pluripotent stem cells (iPS).

The term "nanosystem" is employed to designate the physical, chemical or biological material to which the ligand is linked to, either being the nanostructure or enclosing a drug or a combination of drugs to be targeted to a specific cell. Examples include "nanostructures" such as, but are not restricted to, quantum dots, magnetic particles, gold nanoparticles, nanoshells, carbon nanotubes, liposomes, virus containing an agent or a nucleic acid, polymeric particles such as microcapsules, biodegradable microdevices, agarose, gelatin, or other biological or inert material, and the like. The preferred nanosystem is a liposome composed of but not limited to fully hydrogenated soy phosphatidylcholine (HSPC), methoxy-polyethylene glycol phosphatidylethanolamine (DSPE-mPEG, maleimide-polyethylene glycol phosphatidylethanolamine (DSPE-PEGmaleimide), N-methylpalmitoyloleoylphosphatidylcholine (MPOPC), phosphatidylserine (PS), phosphatidylcholine (PC), palmitoyloleoylphosphatidylcholine (POPC), dipalmitoylphosphatidylcholine (PPC), distearoylphosphatidylcholine (DSPC), diphytanoylphosphatidylcholine (DPhPC), sphingomyelin (SM), phosphatidylglycerol (PG), dioleoylphosphatidylethanolamine (DOPE), N-acetoyl-D-erythro-sphingosine (C2-Ceramide), N-butyroyl-D-erythro-sphingosine (C4-Ceramide), N-hexanoyl-D-erythro-sphingosine (C6-Ceramide), N-octanoyl-D-erythro-sphingosine (C8-Ceramide), N-decanoyl-D-erythro-sphingosine (C10-Ceramide), N-lauroyl-D-erythro-sphingosine (C12-Ceramide), N-myristoyl-D-erythro-sphingosine (C14-Ceramide), N-palmitoyl-D-erythro-sphingosine (C16-Ceramide), 3β-hydroxy-5-cholestene-3-hemisuccinate (CHEMS), cholesterol (CHOL) or a combination thereof. The terms "enclosed", "enclosing" and "carrying" refer to the entrapment and/or protection of the agents within the nanosystem as follows, but not limited to: the drug might be adsorbed, associated or anchored at the surface of the nanosystem; the drug can be integrated in the membrane of a lipidic system, such as, but not limited to, liposomes; and the drug can be entrapped in the inner core of the nanosystem.

As mentioned herein, the term "ligand" and "targeting ligand" designates the molecule linked to the nanosystem that provides the capacity to preferentially direct the nanosystem to a target and, simultaneously, promote intracellular delivery of the nanosystem. The targeting ligand of the invention can be a protein, a growth factor, a peptide, an aptamer, an antibody, nanobody or a fragment thereof. Ligands and corresponding targets include anti-CD19, which targets CD19 on lymphoma and multiple myeloma cells (Sapra,'04); anti-HER2-Fab', which targets HER2 receptor; cetuximab, that binds to epidermal growth factor receptor (EGFR) (Pan,'07); anti-SSEA1, which binds to SSEA1 surface marker of mESC cells; anti-SSEA3 and 4, that bind to SSEA3 and 4 surface markers of hESC cells, respectively; anti-TRA-1-60 and anti-TRA-1-81 which target the keratin sulfate antigens TRA-1-60 and TRA-1-81 in ES cells, respectively (Adewumi,'07); anti-GCTM2 and anti-GCT343, that target GCTM2 and GCT343 antigens in ESC cells, respectively (Adewumi,'07); anti-CD9, anti-Thy1 and anti-HLA1, which bind to surface protein antigens CD9, Thy1 and HLA1 in ES cells, respectively (Adewumi,'07); anti-CD44, anti-CD133 and anti-EpCAM, which target the CD44, CD133 and EpCAM surface antigens expressed in cancer stem cells (Visvader,'08)], any ligand which targets nucleolin, including, but not limited to, endostatin (Zhuo,'10), HB-19 and related Nucant pseudopeptides (Krust,'11), N6L (Birmpas,'12) and F3 peptide (KDEPQRRSARLSAKPAPPKPEPKPKKAPAKK) (SEQ ID NO:1) (Christian,'03). Other targets include neuropilin 1, neuropilin 2, sigma receptor, PSMA (prostate specific membrane antigen), CD24, CD34, CD38 and vascular endothelial growth factor receptor, pituitary adenylate cyclase-activating peptide receptors, lymphatic vessel endothelial hyaluronan receptors, prohibitin, thrombin receptor protease-activated receptor type 1, Notch, integrin receptors, lectin receptor, lactoferrin receptor, annexin 1, platelet-derived growth factor receptors, ephrin receptors as, but not limited to, the ephrinA4 receptor, C-kit, glycoprotein 60, aminopeptidase A, aminopeptidase N, CD13, endosialin, plectin-1, p32/gC1q receptor, fibronectin ED-B, fibrin-fibronectin complexes, interleukin 11 receptor alpha, protease-cleaved collagen IV, hyaluronic binding protein, NG2 proteoglycan, prohibitin, heat shock protein 90, matrix metalloproteinase 2, matrix metalloproteinase 9. The term "peptide" is broadly used to describe peptides, protein fragments, peptide-like molecules, chemically modified peptides or protein fragments, peptides or protein fragments containing non-naturally occurring aminoacids, peptoids and similar structures which bind preferentially a target.

The targeting ligand can be covalently of non-covalently linked or attached to the support. The attachments can be direct or indirect. In embodiments involving indirect attachment, the targeting ligand is attached to a spacer which in turn is attached to the support. Such spacers can be positioned between the targeting ligand and the support such that the interaction of the ligand with the target is not hindered. The nanosystem herein provided, therefore, can present at its surface an engraftment of the ligand in a way that allows the interaction of the specific sequence with the target molecule which may be overexpressed on the surface of a target cell. The ligand engraftment can be accomplished by, but not limited to, conjugation with a PEG modified lipid, for instance DSPE-PEG, bearing a suitable functional group at the terminus of the PEG chain. This modified PEG-lipid can either be an integrant part of the nanosystem or may be coupled to the ligand apart from the nanosystem, aiming at further insertion onto the membrane by the post-insertion procedure. Other spacers include, but not limited to, carbon spacers typically between 0 and 20 carbons long, more typically between 1 and 10 carbons long, which can be represented by R-(CH₂)ₙ-R', wherein "n" can be, independently for each of R and R', any "n" length (e.g., n= 0, 1, 2, 3, 4, 5, 6, 7, 8, 9, 10, 11, 12, 13, 14, 15, 20, 25, etc.); 2-iminothiolane-derived ligands linked by thioether bond to PEG-maleimide; pyridylditiopropionoylamino (PDP)-PEG, hydrazide (Hz)-PEG, or p-nitrophenylcarbonyl (pNP)-PEG dependent chemistry can also be used for spacing ligands and PEG.

The ligand causes the nanosystem home to a specific organ, tissue and the like, by preferentially and/or specifically binding to target molecule carried on the surface of a cell. By preferentially bind it is meant that the ligand binds the target as well as other molecules, but preferentially binds the target versus the other molecules. By specifically bind, it is meant that the ligand binds only the target and not other molecules to any significant degree in physiological conditions. By preferentially deliver it is meant deliver to the target cell relative to other cells to a degree permitting the therapeutic or diagnostic use intended. By specifically deliver it is meant deliver to a target cell and not to other cells to any significant degree in physiological conditions. According to the invention, the targeting ligand selectively binds a tumor cell. As used herein, the terms "organ" and "tissue" are employed to designate an organ or a tissue to which the ligand homes to. They encompass, but are not limited to, diseased organs or tissues, such as a tumor, and naturally or artificial engineered tissues derived from stem cells upon oriented differentiation to specific cell type promoted by targeted delivery of adequate molecules conveyed by the described targeted nanosystem. The term "tumor" includes all related cells present in the tumor microenvironment, including cancer cells and cancer stem cells. The term "cancer stem cells" designates a subset of tumor cells with stem-like characteristics, such as self-renewal, able to sustain tumor growth and responsible for tumor refractoriness and with increased drug resistance. The term "cancer cells" designates a bulk of tumor cells with limited capacity to sustain tumor growth. The term "specific cell type" designates a differentiated somatic cell able to perform the functions with similar capacity of a naturally occurring somatic cell of the respective tissue.

The terms "synergistic", "non-synergistic", "additive" or "antagonistic" are employed to designate the nature of the interaction between drugs as a result of the combination of their individual effects and measured by available quantitative methods or algorithms.

The present disclosure provides a system composed of a nanosystem, which may act as the agent or enclosing an agent or a synergistic or non-synergistic combination of agents, linked to a ligand that preferentially and/or specifically targets overexpressed molecules on target cells, aiming at diagnose, treat, and correct a disease and/or disorder. Additionally, the same system allows for stem cell monitoring or to deliver an agent or a synergistic or non-synergistic combination of agents to modify, interfere, and/or promote oriented stem cell differentiation.

### Technological evolution

Stem cell-based therapies hold a great promise in managing several human diseases. Nonetheless, tumor cells with stem-like features, known as cancer stem cells, present themselves as main propellers of tumor development and recurrence (Visvader,'08). Therefore, one could actually collect increased benefit from specific targeted delivery approaches aiming at either stem cells or cancer stem cells. Indeed, the ability to target cancer stem cells either on solid or hematologic tumors represent a major breakthrough in cancer treatment due to the mentioned intrinsic properties of this particular tumor cell population. Specific and preferential targeting to each cell type can be reached by selecting suitable surface antigens, recognized by a ligand and preferentially promoting cell internalization.

The term "agent" is employed to designate a nanostructure or a drug expected to either allow the acquisition of information without significant cell function modification or to significantly alter the function or status of cells to which it is delivered to.

As used herein, the term "drug" designates a polymeric or a non-polymeric organic chemical, a nucleic acid or an oligonucleotide, a peptide, protein, antibody, growth factor or a fragment thereof presenting a linear or cyclic conformation, a lipid such as a pro-apoptotic lipid, including, but not limited to, the bioactive ceramides, and other non-naturally occurring compounds.

According to the present disclosure, the provided nanoplatform, composed of a nanosystem linked to a ligand, is able to specifically and/or preferentially deliver an enclosed agent or a combination of agents to a specific set of cells when administered to a subject, either for cancer treatment and diagnostics or cell-based therapies. That agent can be, but is not limited to, the chemotherapeutic drug, such as doxorubicin (DXR), a cornerstone in cancer treatment (Minotti,'04). Nonetheless, other drugs for treating cancer have been successfully used such as, but not limited to: alkylating drugs (i.e. cyclophosphamide, chlorambucil melphalan, busulfan, lomustine, cannustine, chlornlethine, estramustine, treosulfan, thiotepa, mitobronitol), cytotoxic antibiotics (i.e. doxorubicin, epirubicin, aclarubicin, idarubicin, daunorubicin, mitoxantrone, bleomycin, dactinomycin and mitomycin), antimetabolites (i.e. methotrexate, capecitabine, cytarabine, fludarabine, cladribine, gemcitabine, fluorouracil, raltitrexed, mercaptopurine, tegafur and tioguaninc), vinca alkaloids (i.e. vinblastine, vincristine, vindesine, vinorelbine, etoposide), platinum compounds (i.e. carboplatin, cisplatin, oxiplatin), taxanes (i.e. docetaxel, paclitaxel), topoisomerase I inhibitors (i.e. irinotecan, topotecan and camptothecin), estrogen receptor antagonists (i.e. raloxifene, tamoxifen, toremifene, lasofoxifene), anti-inflamatory drugs (i.e. ibuprofen, aceclofenac, acemetacin, azapropazone, celecoxib, dexketoprofen, diclofenac, diflunisal, cetodolac, fenbufen, fenoprofen, flubiprofen, indomethacin, acetaminocin, piroxicam, rofecoxib, sulindac, tenoxicam, tiaprofenuic acid, aspirin and benorilate), quinolones (including, but not limited to, ciprofloxacin and trovafloxacin), tyrosine kinase inhibitors (TKI), (including, but not limited to, imatinib, dasatinib, nilotinib, bosutinib, lapatinib, gefitinib, erlotinib, vandetanib, vemurafenib, crizotinib, sorafenib, sunitinib, pazopanib, regorafenib, cabozantinib), berberine and pro-apoptotic lipids (e.g. but not limited to, retinoic acid, all-trans retinoic acid and ceramides, including C2-ceramide, C4-Ceramide, C8-Ceramide, C10-Ceramide, C12-Ceramide, C14-Ceramide or C16-Ceramide).

Furthermore, cancer stem cell modulators can be employed, including, but not limited to, metformin and retinoic acid (lliopoulos,'11; Li,'11). Additionally, the nanosystem of the present invention can be modified, as shown in a preferred example, in order to accommodate pro-apoptotic lipids, including, but not limited to, the bioactive ceramides (Hannun,'08). Nonetheless, modulators of sphingolipid metabolism can also be enclosed in the nanosystem, including ceramidase (CDase) inhibitors (i.e. desipramine, NOE, B13, LCL385, D-e-MAPP) or glucosylceramide synthase (GCS) inhibitors, such as PPMP (Gouaze,'05; Canals,'11).

In embodiments, the agent can be a label. Examples of suitable labels include, but are not limited to, quantum dots, gold nanoshells, paramagnetic particles and fluorophores.

In order to circumvent the drug resistance phenomenon, to which cancer stem cells have been associated due to intrinsic resistance properties, each of the mentioned agents can be used in combination at specific ratios to enable a synergistic effect. In a preferred example, free doxorubicin has been combined with the pro-apoptotic lipid C6-ceramide, which targets the PI3k/Akt pathway (Patwardhan,'11), at specific ratios, demonstrating a synergistic effect against the two cancer cell lines tested (EXAMPLE 1). We have encountered a way to translate this information into the *in vivo* setting, securing that the selected drug ratio reaches the tumor by encapsulation into a tumor-targeted nanocarrier. Mayer *et al.* demonstrated that controlling synergistic ratios of irinotecan/floxuridine or cisplatin/daunorubicin, upon encapsulation into liposomes, improved therapeutic activity in tumor-bearing mice when compared to antagonistic ratios, after systemic administration (Mayer,'06). The same rationale was employed by others to encapsulate a combination of mitoxantrone/imatinib (Pinto,'11). Nonetheless, this approach lacks specificity because it is based on the use of non-targeted liposomes, which rely only on the enhanced permeability and retention effect to accumulate at the tumor site. Aiming at further promoting increased intracellular accumulation of the chosen drug combination into the target cells, nanosystems, such as liposomes, can be armed with specific internalizing targeting moieties.

The present invention, in its preferred embodiment, provides a liposomal nanosystem targeted by the F3 peptide (KDEPQRRSARLSAKPAPPKPEPKPKKAPAKK) (SEQ ID NO:1), which enables the intracellular delivery of a single or combination of drugs in a specific and cell-dependent manner. In one example (EXAMPLE 2), the invention provides a liposomal system covalently coupled to F3 peptide, which targets cell surface internalizing nucleolin. The nanoplatform is capable of retaining and deliver a synergistic drug combination of doxorubicin and C6-ceramide, a pro-apoptotic lipid that targets the PI3K/Akt pathway (Patwardhan,'11). Characterization studies demonstrate that the presence of ceramide has minimal impact in the physicochemical properties of the targeted liposomes containing the drug combination, when compared to the counterpart formulation, without ceramide (EXAMPLE 2). Nonetheless, increased cytotoxicity against breast cancer cell lines was achieved *in vitro* by the targeted formulations containing the drug combination when compared to controls (EXAMPLE 3), thus supporting that intracellular delivery of drug combinations enables increased therapeutic potential when compared to single drug use. This is a relevant outcome when dealing with drug resistance, to which cancer stem cells represent a strong contributor, besides their capability to fuel tumor development (Visvader,'08).

In one non-claimed aspect of the present disclosure (EXAMPLE 4), surprisingly it has been demonstrated that rhodamine-labelled F3-targeted nanosystem binds to and is internalized by embryonic stem cells in a ligand specific manner. This demonstrates indirectly that surface nucleolin was expressed in normal embryonic stem cells, thus providing a valuable target for therapeutic intervention. These results thus pave the way to specific agent delivery to these cells, giving rise to a wide variety of applications, especially in the field of cell-based therapies. The system proposed in this invention can be administered systemically to a subject in order to preferentially and/or specifically deliver its payload to stem cells or it can be delivered as a part of a 3-dimensional scaffold, acting as controlled release system and thus enhancing drug delivery. The term "3-dimensional scaffold" means a three dimensional structure, either of organic or synthetic nature, in which stem cells can be deposited and receive specific stimuli, envisioning regenerative medicine applications. Several agents that modulate cell fate can be enclosed in the proposed system including, but not limited to, VPA, NaB, BIX-01294, parnate, RG108, PD0325901, PD98059, A38-01, SB431542, CHIR99021, Kenpaullone, BayK 8644, Vitamine C, Thiazovivin, Y-27632, apigenin, E-616452, EI-275, Forskolin, PS48, LY294002, stauprimide, IDE1, indolactam V, StemRegenin1, AMD3100, diprotin A (Zhu,'11).

In another example (EXAMPLE 5), cellular association of rhodamine-labeled F3-targeted liposomes with cancer stem cells was assessed by flow cytometry, upon the identification of CSC in cell line models as previously described (Croker,'09). The results demonstrated that targeted liposomes associated with all identified cell populations in a ligand-specific manner, and were internalized by an active process. Most strikingly, cells expressing high levels of both markers (cancer stem cells) presented higher cellular association than cells expressing lower levels of each marker (non-cancer stem cells or cancer cells), a phenomenon that was F3 peptide-dependent. These results, together with the demonstration of increased cytotoxicity by intracellular delivery of drug combinations (EXAMPLE 3), create the opportunity to overcome the drug resistant phenotype associated with cancer stem cells. Overall, the generated results demonstrated that the system of the present invention is preferentially internalized by cancer stem cells, thus allowing specific targeting to this important cell population in the tumor microenvironment, without neglecting other tumor cells. Furthermore, in a non-claimed embodiment, it has been clearly demonstrated that the nucleolin-targeted system was also internalized by embryonic stem cells, demonstrating the presence of surface nucleolin in stem cells, thus enabling the system to be used in agent delivery to these cells. Additionally, the properties of the system allow it to maintain a combination of agents overtime, a feature that, together with the targeting capacity, renders possible to deliver at the intracellular level a combination of drugs, either to enhance cytotoxic effect over target cells for cancer treatment or to promote modification or monitoring of stem cells.

### EXAMPLES

The following set of examples is intended to illustrate the present invention.p[F3]ᵢDC11 and p[F3]ᵢDC12 are according to the invention; the other liposomal formulations are for reference.

### EXAMPLE 1 (reference)

### - Design of drug synergistic combinations of doxorubicin and C6-ceramide

### Cell culture

MDA-MB-231 and MDA-MB-435S breast cancer cell lines were cultured in RPMI 1640 (Sigma) supplemented with 10% (v/v) of heat-inactivated Fetal Bovine Serum (FBS) (Invitrogen), 100 U/ml penicillin, 100 µg/ml streptomycin (Lonza) and maintained at 37°C in a 5% CO₂ atmosphere.

### Evaluation of the interaction between DXR and C6-ceramide

Serial dilutions of doxorubicin or C6-Ceramide (in DMSO), alone or in combination, at fixed molar ratios, were incubated with 8000 MDA-MB-231 or MDA-MB-435S breast cancer cells/well, for 24 h at 37°C in an atmosphere of 5% CO₂. Final DMSO concentration was below 2% (v/v) and had a minimal impact in cell viability (data not shown). Following incubation, cell culture medium was exchanged for fresh one and the experiment was further prolonged between 24 h and 72 hCell viability was then evaluated using the MTT assay as previously described (Moreira,'02).

Data generated from the *in vitro* screening were analyzed using the median-effect method described by Chou and Talalay (Chou,'06; Chou,'11). With this method, the combination index (CI), a measurement of the nature of interaction between the two drugs, has been determined. A combination index of <1, ≈1 or >1, corresponds to a synergistic, additive or antagonistic interaction, respectively.

To assess the interaction between the two drugs, different doxorubicin (DXR):C6-ceramide (C6-Cer) ratios, from 1:40 to 5:1, were tested, using the MDA-MB-231 and MDA-MB-435S breast cancer cell lines. These data indicated (Figure 1) that the combination of doxorubicin and the apoptotic lipid C6-ceramide generated synergistic effects against breast cancer cells, at specific drug ratios.

### EXAMPLE 2 - Preparation and characterization of lipid-based nanosystems

This example provides methods for the preparation of lipid-based nanosystems enclosing an agent and for attaching a targeting ligand onto its surface.

pH-sensitive liposomes without ceramide were composed of 1,2-dioleoyl-sn-glycero-3-phosphoethanolamine; 3β-hydroxy-5-cholestene-3-hemisuccinate; 1,2-distearoyl-sn-glycero-3-phosphocholine; cholesterol; 1,2-distearoyl-sn-glycero-3-phosphoethanolamine-N-[methoxy(polyethylene glycol)-2000] (37:19:19:19:7 mol%).

pH-sensitive liposomes incorporating ceramide were composed of 1,2-dioleoyl-sn-glycero-3-phosphoethanolamine; 3β-hydroxy-5-cholestene-3-hemisuccinate; 1,2-distearoyl-sn-glycero-3-phosphocholine; cholesterol; 1,2-distearoyl-sn-glycero-3-phosphoethanolamine-N-[methoxy(polyethylene glycol)-2000]; N-hexanoyl-D-erythro-sphingosine (37:19:9:9:7:19 mol%). Alternatively, N-hexanoyl-D-erythro-sphingosine was replaced by either N-octanoyl-D-erythro-sphingosine or N-palmitoyl-D-erythro-sphingosine, maintaining molar ratio.

Dried lipid films (lipid film hydration method) or, alternatively, ethanol solutions containing each lipid formulation (ethanol injection method) were hydrated at 50°C - 65°C with ammonium sulfate solution, ranging from pH 7.0 to pH 9.0. Liposomes were extruded through 50 nm - 200 nm pore size polycarbonate membranes using a LiposoFast Basic mini extruder (Avestin, Canada). The buffer was exchanged in a Sephadex G-50 gel column (Sigma) equilibrated with Trizmabase sucrose buffer (pH 7.0 to pH 9.0). Encapsulation of DXR was carried out through ammonium gradient method, upon incubation with liposomes for 1 to 4 h at temperatures ranging from 50°C to 65°C. Non-encapsulated DXR was separated using a Sephadex G-50 gel column equilibrated with 25 mM HEPES, 140 mM NaCl buffer (pH 7.0 to 9.0). To further prepare targeted liposomes, DSPE-PEG₂ₖ-F3 and DSPE-PEG₂ₖ-NS conjugates were prepared. Briefly, thiolated derivatives of F3 (KDEPQRRSARLSAKPAPPKPEPKPKKAPAKK) (SEQ ID NO:1) peptide and non-specific ligand (ARALPSQRSR) (SEQ ID NO:2) (Genecust, Luxemburg) were generated by reaction at room temperature with 2-iminothiolane (Sigma) in 25 mM HEPES, 140 mM NaCl, 1 mM EDTA buffer (pH 7.0 to pH 8.5) for 1 h in an inert N₂ atmosphere. Thiolated derivatives were then incubated overnight at room temperature with DSPE-PEG₂ₖ-maleimide (1,2-distearoyl-sn-glycero-3-phosphoethanolamine-N-[maleimide(polyethylene glycol)-2000]) micelles in 25 mM HEPES, 25 mM MES, 140 mM NaCl, 1 mM EDTA (pH 7.0 to pH 8.0). Micelles were then added to pre-formed liposomes, at 1 mol% to 6 mol% relative to total lipid (TL), and DSPE-PEG₂ₖ-Peptide conjugates post-inserted onto the liposomal membrane upon incubation for 1 h to 15h at temperatures ranging from 37°C to 55°C.

For preparation of calcein loaded liposomes, both lipid films were instead hydrated with a calcein solution and extruded as described above. Afterwards, the calcein excess was removed trough a Sephadex-G50 column equilibrated with 25 mM HEPES, 140 mM NaCl buffer (pH 7.0 to 9.0), and liposomes were immediately submitted to the post-insertion procedure as described.

Liposome size and polidispersion index (PDI) were measured by light scattering with a N5 particle size analyzer (Beckman Coulter, California, US). Final total lipid concentrations were determined upon quantification of cholesterol using Infinity® Cholesterol kit (ThermoScientific, Massachusetts, US). Encapsulated doxorubicin was assayed at 492 nm from a standard curve, after liposomal solubilization with 90% of absolute ethanol, and the loading efficiency (%) was calculated from the equation [(DXR/TL)_{final}/(DXR/TL)ᵢₙᵢₜᵢₐₗ]x100.

To assess drug retention, an aliquot of F3-targeted and non-targeted liposomes, encapsulating either DXR or a combination of DXR and C6-Cer, was incubated in 90% HEPES buffer saline pH 7.4 (HBS), 90% of RPMI 1640 culture medium (Sigma) supplemented with 10% of FBS (Invitrogen) or 90% of FBS, at 37°C. At time 0, 4 and 24 h, DXR fluorescence dequenching was measured in a Spectramax fluorimeter (λₑₓ = 485 nm; λₑₘ = 590 nm) (Molecular Devices, US). Complete release of DXR was attained with 0.25% (v/v) of Triton X-100. Drug retention of DXR (% of control) was calculated using the following formula: 100 - [(TestRFUₙ - MeanRFU₀)/(MeanRFU_{ctr} - MeanRFU₀)]x100, where TestRFUₙ - fluorescence of test at different time points, MeanRFU₀ - fluorescence of test at time 0 h and MeanRFU_{ctr} - fluorescence of complete released sample.

Before proceeding to the loading of the established drug ratio into the pH-sensitive liposomes, the influence of the presence of ceramide in such property was assessed. Liposomes were thus loaded with calcein at a self-quenching concentration as described (Moura,'12). Such concentration enabled one to measure the content release of liposomes inside the cell, since only diluted (dequenched) calcein emitted a fluorescence signal. F3 peptide-targeted liposomes containing calcein with or without C6-ceramide (p[F3]iSL(C6) and p[F3]iSL, respectively) presented a similar extent of intracellular payload release (Figure 2A and 2B), with greater efficiency than the corresponding non-targeted counterparts (pSL(C6) and pSL). Similar result was obtained when liposomes were prepared by ethanol injection method incorporating C8 or C16-ceramide (Figure 8A and 8B).

As shown previously, the DXR:C6-cer 1:2 ratio presented a synergistic interaction between the two drugs, contrasting to the ratio 1:1, which was mildly additive or antagonist depending on the cell line (Figure 1A and 1B). Therefore, F3-peptide targeted (according to the invention) and non-targeted (not according to the invention) liposomes containing the indicated DXR:C6-Cer ratio were prepared by incorporating C6-ceramide in the liposomal bilayer membrane at a fixed molar ratio. Additionally, DXR loading was carried out using the same or half of the quantity of C6-Ceramide presented in the bilayer, thus rendering the DXR:C6-Cer 1:1 or 1:2 molar ratios, respectively.

The data from characterization studies demonstrated that the presence of C6-ceramide in the bilayer of liposomes had minimal impact in loading efficiency, mean size and polydispersion index, when compared to the corresponding targeted (p[F3]ᵢSL - targeted liposomes without ceramide) and non-targeted (pSL - non-targeted liposomes without ceramide) controls (Figures 3A, 3B and 3C). Interestingly, F3 targeted formulations presented lower PDI values, indicating to be more homogenous (Figure 3C). Additionally, incorporation of C8 or C16-ceramide into liposome membrane by ethanol injection significantly decreased targeted liposome size (Figure 7A), which presented also low PDI (Figure7B), with minimal impact on doxorubicin loading efficiency (Figure 7C).

Further evidence of improved stability arouse from data of drug release studies performed at 37°C. At 4 h of incubation, none of the nanosystems tested, either F3-targeted or non-targeted, presented significant drug leakage regardless the medium used. Nonetheless, at 24 h of incubation either in cell culture medium or serum, all formulations, either targeted or non-targeted, presented drug leakage, in a similar extent and more marked in the latter medium (Figures 3E and 3F, respectively). Except for liposomes encapsulating the doxorubicin:C8-ceramide 1:1 ratio in FBS at 37°C, the results were similar as the ones obtained with liposomes containing C6-ceramide and doxorubicin (Figures 7D-7G).

Strikingly, data generated in HBS clearly demonstrated that p[F3]ᵢDC11 (targeted liposomes encapsulating the DXR:C6-Cer 1:1 molar ratio, according to the invention) and p[F3]ᵢDC12 (targeted liposomes encapsulating the DXR:C6-cer 1:2 molar ratio, according to the invention) share the same stability in terms of drug retention when compared to control liposomes (p[F3]ᵢSL), and are more stable than the non-targeted counterparts (pDC11 and pDC12, respectively) (Figure 3D), in accordance with the PDI results (Figure 3C). Additionally, the non-targeted pDC11 and pDC12 formulations, presented increased leakage when compared to the respective control, pSL liposomes (Figure 3D). The removal of DSPC and cholesterol, known to increase drug retention in pH-sensitive liposomes (Ishida,'06), to accommodate the incorporation of C6-ceramide, associated with the increased defects in membrane bilayer promoted by the sphingolipid (Khazanov,'08), was likely the reason behind such difference. Nonetheless, the same data demonstrated that the F3 ligand stabilized the bilayer membrane of C6-ceramide loaded liposomes, to the same extent of liposomes without C6-ceramide (Figure 3D). This might be explained by the additional hydration layer on the liposomal surface, arising from the charged F3 peptide. This effect seems to improve drug retention (Figure 3D) and minimize aggregation (Figure 3C).

Furthermore, the differences observed in the different media, might be related to their differences in the ion composition. Those, in turn, may promote different degrees of lipid headgroup hydration, especially of CHEMS, by providing diverse counterions, which render unequal levels of membrane stabilization (Hafez,'00; Li,'01). This fact could explain the increased drug retention of pDC11 and pDC12 formulations in cell culture medium when compared to HBS buffer.

### EXAMPLE 3 - Overall cytotoxic effect of liposomal formulations

Different concentrations of DXR-containing F3 peptide-targeted (p[F3]ᵢSL, p[F3]ᵢDC11 and p[F3]ᵢDC12) or non-targeted (pSL, pDC11 and pDC12) liposomal formulations were incubated with 8000 MDA-MB-231 or MDA-MB-435S breast cancer cells/well, between 1h and 24 h at 37°C in an atmosphere of 5% of CO₂. Afterwards, cell culture medium was exchanged for fresh one and the experiment was prolonged for a total of 96 h. Cell viability was then evaluated using the MTT assay as described (Moreira,'02).

In order to evaluate the cytotoxic potential, the impact of each formulation on cell viability was assessed. The data demonstrated that the p[F3]iDC11 and p[F3]iDC12 formulations allowed increased cytotoxic effect, enabling an effect over 90% in a 24 h incubation with, at least, a doxorubicin dose 4-fold lower when compared to the standard p[F3]iSL (Figure 4A and 4C, and Table 1). The introduction of DXR:C6-cer combination in non-targeted liposomes also increases their cytotoxicity, an effect, however, that is expected to be absent *in vivo* (Figures 4B and 4C). Nonetheless, the IC₉₀ values for the targeted formulations remained normally half-dose lower than those for the non-targeted counterparts (Table 1).

This increase in activity for the non-targeted liposomes was not unexpected. The incorporation of C6-ceramide to the liposome bilayer, by itself, renders any formulation more potent, either by internalization of the intact liposome by cells or by simple diffusion of the lipid from the liposomal bilayer to the cell membrane (Khazanov,'08). Nevertheless, loss of this advantage is expected to take place *in vivo,* as the absence of the F3 peptide targeting moiety significantly decreases tumor accumulation relative to the targeted counterpart (Moura,'12).

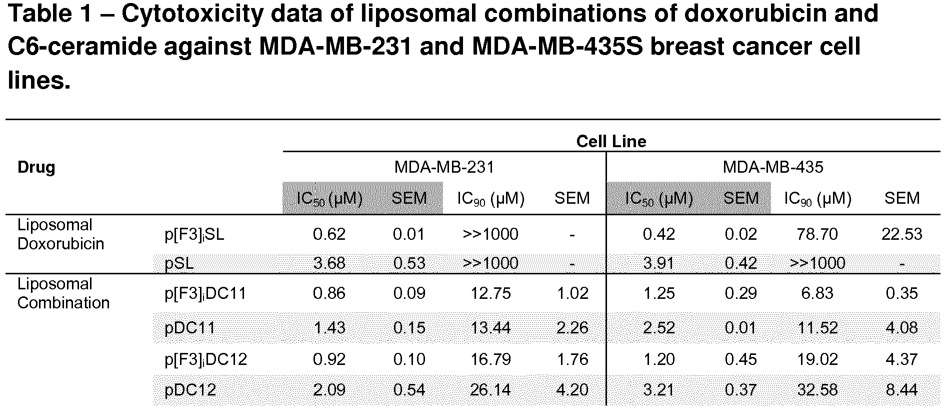

Cells were incubated for 24 h with the liposomal drugs at serially diluted concentrations of DXR, and the experiment was further prolonged for 72 h after which cell death was assessed. The data represent the mean ± SEM of IC₅ₒ or IC₉₀ calculated from 3 independent experiments. Values were calculated using the median-effect equation (Chou,'06).

Nevertheless, C6-ceramide might also provoke defects in the liposomal membrane assembly, which, in turn, might affect the capacity of the membrane to retain any encapsulated drug (Khazanov,'08). In fact, despite not significant, the pDC11 and pDC12 formulations presented decreased drug retention when compared to pSL nanosystem, when incubated in cell culture media (83.1% and 79.6%, respectively, versus 96.9% - Figure 3E), which may also account for the increased cytotoxic effect. Data from characterization studies demonstrated that loading of the selected DXR-C6-ceramide ratios into F3-targeted liposomes imposed minimal impact in terms of stability when compared to F3 targeted standard liposomes (Figure 2). Indeed, the data indicated that the F3-targeted formulations containing the drug combination present a drug retention of 88.0% and 92.1% for p[F3]ᵢDC11 and p[F3]ᵢDC12, respectively (versus 96.5%, p[F3]ᵢSL), thus an effect accounting in lesser extent to the cytotoxic effect. Therefore, the post-insertion of F3 peptide, by further stabilizing the liposome bilayer defects imposed by C6-ceramide and by granting intracellular access of the drug combination, present an increased advantage in their application in vivo. The above results were also reproduced by liposomes prepared by ethanol injection method co-encapsulating DXR:C8-Ceramide ratios 1:1 and 1:2 (Figure 9). Furthermore, the results suggested that the incorporation of both drug combinations into targeted liposomal formulations may change the interaction nature of drug combinations. The free drug combination studies clearly indicated that the DXR:C6-cer 1:2 ratio was synergistic, whereas the 1:1 ratio would be mildly additive or antagonistic (Figure 1). Alternatively, the present data demonstrated that the p[F3]iDC11 formulation is the most active, an effect also dependent on the drug sensitivity of each cell line (Figure 4 and Table 1). This thus suggests that the interaction between drugs might also be dependent on the mechanism of drug delivery, particularly on the ability to delivery intracellularly a specific drug ratio. Nonetheless, it was demonstrated that the encapsulation of the combination at 1:2 ratio (p[F3]iDC12) allows for increased liposomal citotoxicity using half of the drug loading per liposome utilized to prepare the standard formulation (p[F3]ᵢSL). Ultimately, this could prove useful to further increase the safety associated with the use of liposomal doxorubicin.

### EXAMPLE 4 (reference)

### - Cellular association studies with embryonic stem cells

E14 mESC were cultured in KnockOut-DMEM (GIBCO) supplemented with KnockOut Serum Replacement (GIBCO), 1% Pen-Strep (GIBCO), non-essential aminoacids (100 µM) (Sigma), L-glutamine, 0.1 mM β-Mercaptoethanol in the presence of 1000 U/mL of leukemia-inducible factor (LIF) (Millipore) at 37°C in an atmosphere of 5% of CO₂. For the cellular association studies, cells were cultured for 72 h in medium either fully supplemented, maintaining pluripotency status, or in absence of LIF and serum replacement to induce differentiation (conditions under which pluripotency status is lost). Cells were then incubated with 0.1 mM to 0.4 mM of total lipid of each formulation, between 1 h and 4 h, at 4°C or 37°C, either as cell suspension (Figure 5A) or as colonies (Figure 5B). Upon washing, cellular association was analyzed by flow cytometry in FACScalibur system (BD Biosciences, US). Non-viable cells were excluded from the analysis using 7-aminoactinomycin D (7-AAD) (Sigma-Aldrich).

The successful application of the F3-peptide targeted liposomes co-encapsulating a combination of drugs is highly dependent on the capacity of such system to intracellularly deliver its content to target cells. Since this system is dependent on the expression of cell surface nucleolin, the target cells should necessarily express this receptor.

After 72h in culture as described, cellular association experiments were carried out for 1 h with E14 cells in suspension or as colonies. The results obtained surprisingly indicated that the F3-targeted nanosystem (p[F3]iSL) associated extensively with mESC, when compared to the non-targeted or non-specific targeted counterparts, in a ligand-specific manner (Figure 5). Furthermore, when incubation was carried at 4°C (not permissive to endocytosis), the association of F3-targeted nanosystem decreased, indicating that an active process of internalization was occurring, most likely, endocytosis (Figure 5A). Interestingly, cells cultured without LIF still presented similar levels of association as cells cultured in fully supplemented medium (Figure 5A). Strikingly, culturing E14 cells without LIF and serum replacement, has had a significant impact in cellular association, reducing the signal to levels of the non-targeted nanosystem (Figure 5A). These results demonstrate that membrane nucleolin levels decrease according to cell status, decreasing when they are prompted to differentiate. No less interesting was the fact that, even in colony (Figure 5B), F3-targeted nanosystem associated with E14 cells, nonetheless in lower extent when compared to cells in suspension. Such results could be explained by the lower accessibility of the targeted nanosystem to E14 cells in colony as well as by the increase in surface area for the interaction of this system with the cells in suspension. This was reinforced by the improved results obtained in colony in absence of LIF (Figure 5B), since these colonies are smaller thus facilitating the system access.

These results demonstrated that nucleolin is present at the surface of embryonic stem cells playing a similar role in the internalization of liposomes. Importantly, the data pointed nucleolin as a marker of stemness status of stem cells.

### EXAMPLE 5 - Cellular association of F3-targeted liposomes with cancer stem cells

The cancer stem cell model has gained an undertaking importance in the current knowledge of cancer biology (Frank,'10). The reconnaissance of the existence of a small cell population within the tumor microenvironment with stem cell properties which is able to fuel tumor development has shifted the established paradigm of clonal tumor organization, in which cells share the same features, to a hierarchical organization model where at the top rests a population of cells able to self-renew and give rise to more differentiated and committed cells of a tumor environment (Ginestier,'07; Visvader,'08; Visvader,'11). Furthermore, drug resistance is inherently associated with these cells, as well as the activation of different signaling pathways, such as the PI3K/Akt pathway (Visvader,'08; Dubrovska,'09; Korkaya,'09; Croker,'11). Despite the lack of a fully established panel of markers for the identification of cancer stem cells, either due to the variability encountered among different cancers (Stuelten,'10) or to the absence of marker specificity (Clarke,'06), several markers have been employed successfully, such as CD44, CD24 or aldehyde dehydrogenase activity, normally combined with functional assays. These identify subpopulations of cells with stem cell characteristics, which present increased tumorigenic capacity, ability to recapitulate tumor environment and are associated with metastization and poor disease prognosis (Al-Hajj,'03; Ginestier,'07; Morimoto,'09; Marcato,'11). Therefore, therapeutic intervention aiming the cancer stem cell population could bring major benefits.

Cellular association between the F3 peptide targeted liposomes (p[F3]ᵢSL) and the identified populations of cells was assessed as previously described (Croker,'09), in order to understand if one could actually deliver a payload to these cells.

Half-million MDA-MB-231 or MDA-MB-435S breast cancer cells, known to contain functional cancer stem cells (Croker,'11; Marcato,'11), were incubated with rhodamine-labelled F3-peptide targeted or non-targeted liposomes, or liposomes targeted by a non-specific peptide (at 0.1 to 0.4 mM of total lipid), between 1 h and 4 h at 37°C or 4°C. After washing, cells were stained aiming at identifying cancer stem cells, as previously described (Croker,'11). Briefly, cells were first incubated with anti-CD44-PECy5 antibody [rat IM7 clone] (Abcam, UK) or IgG2b isotype control (Biolegend, US) for 30 min at 4°C in PBS buffer with 1 % BSA and 0.1% sodium azide (PBS-BSA). Cells were then washed with PBS-BSA and stained with ALDEFLUOR® kit (StemCell Technologies, Grenoble, FR) for identification of aldehyde dehydrogenase (ALDH) activity according to the manufacturer instructions. The cell-associated rhodamine signal was immediately analyzed by flow cytometry in a FACScalibur system (BD Biosciences, US) and a total of 30,000 events were collected. Appropriate controls were employed to assure correct compensation of fluorescence signals in each channel.

Through population gating, four regions were identified: R1, characterized by low staining for CD44 and high ALDH signal (CD44^{-/low}/ALDH^{hi}); R2 and R3, characterized by high (CD44⁺/ALDH^{hi}) or low (CD44^{-/low}/ALDH^{-/low}) expression of both markers, respectively; and R4, characterized by high CD44 expression and low ALDH activity (CD44⁺/ALDH^{-/low}). These populations were then plotted in Side Scatter(y)-Rhodamine(x) dot plots, and the fluorescence geometric mean of rhodamine of each population was obtained.

Of utmost importance, the CD44⁺/ALDH^{hi} population (enriched for cancer stem cells) presented 64% or 61% higher cellular association when compared to the CD44⁻/ALDH^{-/low} population (non-cancer stem cells) for MDA-MB-231 and MDA-MB-435S respectively, an effect that was dependent on the presence of the F3 peptide (Figures 6A and 6B, respectively). Additionally, F3-peptide targeted liposomes also associated with CD44^{-/low}/ALDH^{hi}, and CD44⁺/ALDH^{-/low} populations, which might represent intermediate stages in the hierarchical organization of the cancer cell lines (Figure 6).

For each of the breast cancer cell line models used, the results clearly indicated that the fluorescently-labeled F3 peptide-targeted liposomes associated with all the identified subpopulations of breast cancer cells in a higher extent and in a ligand-specific manner than non-targeted liposomes (pSL) or liposomes targeted by a non-specific peptide (p[NS]ᵢSL) at 37°C (Figure 6A and 6B). Furthermore, at 4°C, a temperature not permissive to endocytosis, the F3 peptide-targeted liposomes presented lower cellular association with both cell line models, thus indicating that an energy-dependent internalization was taking place (Figure 6).

The above results of cellular association using cancer cell lines as models for identification of a cancer stem cell enriched population (Charafe-Jauffret,'09; Croker,'09; Stuelten,'10), surprisingly showed that, it is possible to target this population using nanotechnological platforms, thus enabling specific intracellular delivery of drug payloads (Figure 7)). Furthermore, the results demonstrated that the increased F3 targeted liposomes cellular association with CD44⁺/ALDH^{hi} population is dependent on cell surface nucleolin, either by increased expression or, most likely, due to increased nucleolin turnover, since, at 4°C, owed to limited cellular activity, the differences between cell subpopulations were not evident anymore (Figure 6). Nonetheless, these results are in accordance with those from cellular association with mESC (Figure 5), thus associating surface nucleolin expression with stem cell and cancer stem cell phenotype.

Overall, the present invention provides a system and methods to preferentially deliver a combination of drugs, at the intracellular level, to cancer stem cells, in the case of cancer condition, enabling therapeutically eradication of a population of cells known to fuel tumor growth and relapse. Additionally, the present invention provides a system and methods to recognize surface nucleolin in stem cells and take advantage of the former to preferentially deliver an agent or a combination of agents to these cells.

### References

Adewumi, O., Aflatoonian, B., Ahrlund-Richter, L., Amit, M., Andrews, P. W., Beighton, G., Bello, P. A., Benvenisty, N., Berry, L. S., Bevan, S., et al. (2007). Characterization of human embryonic stem cell lines by the International Stem Cell Initiative. Nat Biotechnol 25, 803-816.
Al-Hajj, M., Wicha, M. S., Benito-Hernandez, A., Morrison, S. J., and Clarke, M. F. (2003). Prospective identification of tumorigenic breast cancer cells. Proc Natl Acad Sci U S A 100, 3983-3988.
Allen, T. M. (1998). Liposomal drug formulations. Rationale for development and what we can expect for the future. Drugs 56, 747-756.
Barker, N., Ridgway, R. A., van Es, J. H., van de Wetering, M., Begthel, H., van den Born, M., Danenberg, E., Clarke, A. R., Sansom, O. J., and Clevers, H. (2009). Crypt stem cells as the cells-of-origin of intestinal cancer. Nature 457, 608-611.
Birmpas, C., Briand, J. P., Courty, J., and Katsoris, P. (2012). Nucleolin mediates the antiangiogenesis effect of the pseudopeptide N6L. BMC Cell Biol 13, 32*.* Bonnet, D., and Dick, J. E. (1997). Human acute myeloid leukemia is organized as a hierarchy that originates from a primitive hematopoietic cell. Nat Med 3, 730-737.
Boyer, L. A., Lee, T. I., Cole, M. F., Johnstone, S. E., Levine, S. S., Zucker, J. P., Guenther, M. G., Kumar, R. M., Murray, H. L., Jenner, R. G., et al. (2005). Core transcriptional regulatory circuitry in human embryonic stem cells. Cell 122, 947-956.
Canals, D., Perry, D. M., Jenkins, R. W., and Hannun, Y. A. (2011). Drug targeting of sphingolipid metabolism: sphingomyelinases and ceramidases. Br J Pharmacol 163, 694-712.
Charafe-Jauffret, E., Ginestier, C., Iovino, F., Wicinski, J., Cervera, N., Finetti, P., Hur, M. H., Diebel, M. E., Monville, F., Dutcher, J., et al. (2009). Breast cancer cell lines contain functional cancer stem cells with metastatic capacity and a distinct molecular signature. Cancer Res 69, 1302-1313.
Chou, T. C. (2006). Theoretical basis, experimental design, and computerized simulation of synergism and antagonism in drug combination studies. Pharmacol Rev 58, 621-681.
Chou, T. C. (2011). The mass-action law based algorithm for cost-effective approach for cancer drug discovery and development. Am J Cancer Res 1, 925-954.
Christian, S., Pilch, J., Akerman, M. E., Porkka, K., Laakkonen, P., and Ruoslahti, E. (2003). Nucleolin expressed at the cell surface is a marker of endothelial cells in angiogenic blood vessels. J Cell Biol 163, 871-878.
Clarke, M. F., Dick, J. E., Dirks, P. B., Eaves, C. J., Jamieson, C. H., Jones, D. L., Visvader, J., Weissman, I. L., and Wahl, G. M. (2006). Cancer stem cellsperspectives on current status and future directions: AACR Workshop on cancer stem cells. Cancer Res 66, 9339-9344.
Cohen, D. E., and Melton, D. (2011). Turning straw into gold: directing cell fate for regenerative medicine. Nat Rev Genet 12, 243-252.
Croker, A. K., and Allan, A. L. (2011). Inhibition of aldehyde dehydrogenase (ALDH) activity reduces chemotherapy and radiation resistance of stem-like ALDH(hi)CD44 (+) human breast cancer cells. Breast Cancer Res Treat.
Croker, A. K., Goodale, D., Chu, J., Postenka, C., Hedley, B. D., Hess, D. A., and Allan, A. L. (2009). High aldehyde dehydrogenase and expression of cancer stem cell markers selects for breast cancer cells with enhanced malignant and metastatic ability. J Cell Mol Med 13, 2236-2252.
D'Amour, K. A., Agulnick, A. D., Eliazer, S., Kelly, O. G., Kroon, E., and Baetge, E. E. (2005). Efficient differentiation of human embryonic stem cells to definitive endoderm. Nat Biotechnol 23, 1534-1541.
De Miguel, M. P., Fuentes-Julian, S., and Alcaina, Y. (2010). Pluripotent stem cells: origin, maintenance and induction. Stem Cell Rev 6, 633-649.
Dubrovska, A., Kim, S., Salamone, R. J., Walker, J. R., Maira, S. M., Garcia-Echeverria, C., Schultz, P. G., and Reddy, V. A. (2009). The role of PTEN/Akt/PI3K signaling in the maintenance and viability of prostate cancer stem-like cell populations. Proc Natl Acad Sci U S A 106, 268-273.
Evans, M. J., and Kaufman, M. H. (1981). Establishment in culture of pluripotential cells from mouse embryos. Nature 292, 154-156.
Frank, N. Y., Schatton, T., and Frank, M. H. (2010). The therapeutic promise of the cancer stem cell concept. J Clin Invest 120, 41-50.
Gao, J., Chen, K., Xie, R., Xie, J., Yan, Y., Cheng, Z., Peng, X., and Chen, X. (2010). In vivo tumor-targeted fluorescence imaging using near-infrared non-cadmium quantum dots. Bioconjug Chem 21, 604-609.
Gao, J., Zhong, W., He, J., Li, H., Zhang, H., Zhou, G., Li, B., Lu, Y., Zou, H., Kou, G., et al. (2009). Tumor-targeted PE38KDEL delivery via PEGylated anti-HER2 immunoliposomes. Int J Pharm 374, 145-152.
Ginestier, C., Hur, M. H., Charafe-Jauffret, E., Monville, F., Dutcher, J., Brown, M., Jacquemier, J., Viens, P., Kleer, C. G., Liu, S., et al. (2007). ALDH1 is a marker of normal and malignant human mammary stem cells and a predictor of poor clinical outcome. Cell Stem Cell 1, 555-567.
Gomes-da-Silva, L. C., Fonseca, N. A., Moura, V., Pedroso de Lima, M. C., Simoes, S., and Moreira, J. N. (2012). Lipid-Based Nanoparticles for siRNA Delivery in Cancer Therapy: Paradigms and Challenges. Acc Chem Res.
Gouaze, V., Liu, Y. Y., Prickett, C. S., Yu, J. Y., Giuliano, A. E., and Cabot, M. C. (2005). Glucosylceramide synthase blockade down-regulates P-glycoprotein and resensitizes multidrug-resistant breast cancer cells to anticancer drugs. Cancer Res 65, 3861-3867.
Hafez, I. M., and Cullis, P. R. (2000). Cholesteryl hemisuccinate exhibits pH sensitive polymorphic phase behavior. Biochim Biophys Acta 1463, 107-114. Hanahan, D., and Weinberg, R. A. (2011). Hallmarks of cancer: the next generation. Cell 144, 646-674.
Hannun, Y. A., and Obeid, L. M. (2008). Principles of bioactive lipid signalling: lessons from sphingolipids. Nat Rev Mol Cell Biol 9, 139-150.
Iliopoulos, D., Hirsch, H. A., and Struhl, K. (2011). Metformin decreases the dose of chemotherapy for prolonging tumor remission in mouse xenografts involving multiple cancer cell types. Cancer Res.
Immordino, M. L., Dosio, F., and Cattel, L. (2006). Stealth liposomes: review of the basic science, rationale, and clinical applications, existing and potential. Int J Nanomedicine 1, 297-315.
Ishida, T., Okada, Y., Kobayashi, T., and Kiwada, H. (2006). Development of pH-sensitive liposomes that efficiently retain encapsulated doxorubicin (DXR) in blood. Int J Pharm 309, 94-100.
Khazanov, E., Priev, A., Shillemans, J. P., and Barenholz, Y. (2008). Physicochemical and biological characterization of ceramide-containing liposomes: paving the way to ceramide therapeutic application. Langmuir 24, 6965-6980.
Korkaya, H., Paulson, A., Charafe-Jauffret, E., Ginestier, C., Brown, M., Dutcher, J., Clouthier, S. G., and Wicha, M. S. (2009). Regulation of mammary stem/progenitor cells by PTEN/Akt/beta-catenin signaling. PLoS Biol 7, e1000121.
Krust, B., El Khoury, D., Nondier, I., Soundaramourty, C., and Hovanessian, A. G. (2011). Targeting surface nucleolin with multivalent HB-19 and related Nucant pseudopeptides results in distinct inhibitory mechanisms depending on the malignant tumor cell type. BMC Cancer 11, 333.
Lamba, D. A., Karl, M. O., Ware, C. B., and Reh, T. A. (2006). Efficient generation of retinal progenitor cells from human embryonic stem cells. Proc Natl Acad Sci U S A 103, 12769-12774.
Li, R. J., Ying, X., Zhang, Y., Ju, R. J., Wang, X. X., Yao, H. J., Men, Y., Tian, W., Yu, Y., Zhang, L., et al. (2011). All-trans retinoic acid stealth liposomes prevent the relapse of breast cancer arising from the cancer stem cells. J Control Release 149, 281-291.
Li, X., and Schick, M. (2001). Theory of tunable pH-sensitive vesicles of anionic and cationic lipids or anionic and neutral lipids. Biophys J 80, 1703-1711.
Lodi, D., lannitti, T., and Palmieri, B. (2011). Stem cells in clinical practice: applications and warnings. J Exp Clin Cancer Res 30, 9.
Marcato, P., Dean, C. A., Pan, D., Araslanova, R., Gillis, M., Joshi, M., Helyer, L., Pan, L., Leidal, A., Gujar, S., et al. (2011). Aldehyde dehydrogenase activity of breast cancer stem cells is primarily due to isoform ALDH1A3 and its expression is predictive of metastasis. Stem Cells 29, 32-45.
Mayer, L. D., Harasym, T. O., Tardi, P. G., Harasym, N. L., Shew, C. R., Johnstone, S. A., Ramsay, E. C., Bally, M. B., and Janoff, A. S. (2006). Ratiometric dosing of anticancer drug combinations: controlling drug ratios after systemic administration regulates therapeutic activity in tumor-bearing mice. Mol Cancer Ther 5, 1854-1863.
Mayer, L. D., and Janoff, A. S. (2007). Optimizing combination chemotherapy by controlling drug ratios. Mol Interv 7, 216-223.
McLean, A. B., D'Amour, K. A., Jones, K. L., Krishnamoorthy, M., Kulik, M. J., Reynolds, D. M., Sheppard, A. M., Liu, H., Xu, Y., Baetge, E. E., and Dalton, S. (2007). Activin a efficiently specifies definitive endoderm from human embryonic stem cells only when phosphatidylinositol 3-kinase signaling is suppressed. Stem Cells 25, 29-38.
Minotti, G., Menna, P., Salvatorelli, E., Cairo, G., and Gianni, L. (2004). Anthracyclines: molecular advances and pharmacologic developments in antitumor activity and cardiotoxicity. Pharmacol Rev 56, 185-229.
Mooney, D. J., and Vandenburgh, H. (2008). Cell delivery mechanisms for tissue repair. Cell Stem Cell 2, 205-213.
Moreira, J. N., Ishida, T., Gaspar, R., and Allen, T. M. (2002). Use of the post-insertion technique to insert peptide ligands into pre-formed stealth liposomes with retention of binding activity and cytotoxicity. Pharm Res 19, 265-269.
Morimoto, K., Kim, S. J., Tanei, T., Shimazu, K., Tanji, Y., Taguchi, T., Tamaki, Y., Terada, N., and Noguchi, S. (2009). Stem cell marker aldehyde dehydrogenase 1-positive breast cancers are characterized by negative estrogen receptor, positive human epidermal growth factor receptor type 2, and high Ki67 expression. Cancer Sci 100, 1062-1068.
Moura, V., Lacerda, M., Figueiredo, P., Corvo, M. L., Cruz, M. E., Soares, R., de Lima, M. C., Simoes, S., and Moreira, J. N. (2012). Targeted and intracellular triggered delivery of therapeutics to cancer cells and the tumor microenvironment: impact on the treatment of breast cancer. Breast Cancer Res Treat 133, 61-73.
Pan, X., Wu, G., Yang, W., Barth, R. F., Tjarks, W., and Lee, R. J. (2007). Synthesis of cetuximab-immunoliposomes via a cholesterol-based membrane anchor for targeting of EGFR. Bioconjug Chem 18, 101-108.
Patwardhan, G. A., and Liu, Y. Y. (2011). Sphingolipids and expression regulation of genes in cancer. Prog Lipid Res 50, 104-114.
Peer, D., Karp, J. M., Hong, S., Farokhzad, O. C., Margalit, R., and Langer, R. (2007). Nanocarriers as an emerging platform for cancer therapy. Nat Nanotechnol 2, 751-760.
Pinto, A. C., Moreira, J. N., and Simoes, S. (2011). Liposomal imatinib-mitoxantrone combination: formulation development and therapeutic evaluation in an animal model of prostate cancer. Prostate 71, 81-90.
Puri, M. C., and Nagy, A. (2012). Concise review: Embryonic stem cells versus induced pluripotent stem cells: the game is on. Stem Cells 30, 10-14. Ramaswamy, S. (2007). Rational design of cancer-drug combinations. N Engl J Med 357, 299-300.
Reya, T., Morrison, S. J., Clarke, M. F., and Weissman, I. L. (2001). Stem cells, cancer, and cancer stem cells. Nature 414, 105-111.
Sapra, P., and Allen, T. M. (2003). Ligand-targeted liposomal anticancer drugs. Prog Lipid Res 42, 439-462.
Sapra, P., Moase, E. H., Ma, J., and Allen, T. M. (2004). Improved therapeutic responses in a xenograft model of human B lymphoma (Namalwa) for liposomal vincristine versus liposomal doxorubicin targeted via anti-CD19 IgG2a or Fab' fragments. Clin Cancer Res 10, 1100-1111.
Schroeder, A., Heller, D. A., Winslow, M. M., Dahlman, J. E., Pratt, G. W., Langer, R., Jacks, T., and Anderson, D. G. (2012). Treating metastatic cancer with nanotechnology. Nat Rev Cancer 12, 39-50.
Shi, Y., Desponts, C., Do, J. T., Hahm, H. S., Scholer, H. R., and Ding, S. (2008). Induction of pluripotent stem cells from mouse embryonic fibroblasts by Oct4 and Klf4 with small-molecule compounds. Cell Stem Cell 3, 568-574.
Simard, P., and Leroux, J. C. (2010). In Vivo Evaluation of pH-Sensitive Polymer-Based Immunoliposomes Targeting the CD33 Antigen. Mol Pharm.
Simoes, S., Moreira, J. N., Fonseca, C., Duzgunes, N., and de Lima, M. C. (2004). On the formulation of pH-sensitive liposomes with long circulation times. Adv Drug Deliv Rev 56, 947-965.
Stuelten, C. H., Mertins, S. D., Busch, J. I., Gowens, M., Scudiero, D. A., Burkett, M. W., Hite, K. M., Alley, M., Hollingshead, M., Shoemaker, R. H., and Niederhuber, J. E. (2010). Complex display of putative tumor stem cell markers in the NCI60 tumor cell line panel. Stem Cells 28, 649-660.
Takahashi, K., Tanabe, K., Ohnuki, M., Narita, M., Ichisaka, T., Tomoda, K., and Yamanaka, S. (2007). Induction of pluripotent stem cells from adult human fibroblasts by defined factors. Cell 131, 861-872.
Takahashi, K., and Yamanaka, S. (2006). Induction of pluripotent stem cells from mouse embryonic and adult fibroblast cultures by defined factors. Cell 126, 663-676.
Tesar, P. J., Chenoweth, J. G., Brook, F. A., Davies, T. J., Evans, E. P., Mack, D. L., Gardner, R. L., and McKay, R. D. (2007). New cell lines from mouse epiblast share defining features with human embryonic stem cells. Nature 448, 196-199.
Thomson, J. A., Itskovitz-Eldor, J., Shapiro, S. S., Waknitz, M. A., Swiergiel, J. J., Marshall, V. S., and Jones, J. M. (1998). Embryonic stem cell lines derived from human blastocysts. Science 282, 1145-1147.
Vallier, L., Mendjan, S., Brown, S., Chng, Z., Teo, A., Smithers, L. E., Trotter, M. W., Cho, C. H., Martinez, A., Rugg-Gunn, P., et al. (2009). Activin/Nodal signalling maintains pluripotency by controlling Nanog expression. Development 136, 1339-1349.
Visvader, J. E. (2011). Cells of origin in cancer. Nature 469, 314-322.
Visvader, J. E., and Lindeman, G. J. (2008). Cancer stem cells in solid tumours: accumulating evidence and unresolved questions. Nat Rev Cancer 8, 755-768. Wang, A. Z., Langer, R., and Farokhzad, O. C. (2012). Nanoparticle delivery of cancer drugs. Annu Rev Med 63, 185-198.
Zhu, S., Wei, W., and Ding, S. (2011). Chemical strategies for stem cell biology and regenerative medicine. Annu Rev Biomed Eng 13, 73-90.
Zhuo, W., Luo, C., Wang, X., Song, X., Fu, Y., and Luo, Y. (2010). Endostatin inhibits tumour lymphangiogenesis and lymphatic metastasis via cell surface nucleolin on lymphangiogenic endothelial cells. J Pathol 222, 249-260.

## Claims

1. A ligand-targeted delivery system comprising,
a targeting ligand that selectively binds a tumor cell linked to a support carrying an agent,
wherein said support is a pH sensitive liposome having a cytotoxic ceramide analog encapsulated, entrapped or intercalated in the support,
wherein the agent is doxorubicin encapsulated in the support,
wherein the molar ratio of cytotoxic ceramide analog to doxorubicin is between 3:1 and 1:2,
wherein said liposome is capable of the pH dependent intracellular release of said cytotoxic ceramide analog and said agent, and wherein the cytotoxic ceramide analog is N-acetoyl-D-erythro-sphingosine, N-butyroyl-D-erythro-sphingosine, N-hexanoyl-D-erythro-sphingosine, N-octanoyl-D-erythro-sphingosine, N-decanoyl-D-erythro-sphingosine, N-lauroyl-D-erythro-sphingosine, N-myristoyl-D-erythro-sphingosine, or N-palmitoyl-D-erythro-sphingosine.

2. The delivery system of claim 1, wherein the cytotoxic ceramide analog is C6-Ceramide and the molar ratio of C6-ceramide to doxorubicin is less than 2:1, is between 1.5:1 and 1:1.5 or is about 1:1.

3. The delivery system of claim 1, wherein the targeting ligand binds a cancer stem cell.

4. The delivery system of claim 1, wherein the targeting ligand (i) binds nucleolin or (ii) is a peptide comprising the amino acid sequence of SEQ ID NO:1.

5. The delivery system of claim 1, wherein the targeting ligand is linked to the support by a spacer positioned between the targeting ligand and the support such that the interaction of the ligand with the target is not hindered.

6. The delivery system of claim 1, wherein the support is a liposome comprising 1,2-dioleoyl-sn-glycero-3-phosphoethanolamine; 3β-hydroxy-5-cholestene-3-hemisuccinate; 1,2-distearoyl-sn-glycero-3-phosphocholine; cholesterol; 1,2-distearoyl-sn-glycero-3-phosphoethanolamine-N-[methoxy(polyethylene glycol)-2000]; and cytotoxic ceramide analog.

7. The delivery system of claim 1, wherein the support is a liposome comprising 1,2-dioleoyl-sn-glycero-3-phosphoethanolamine; 3β-hydroxy-5-cholestene-3-hemisuccinate; 1,2-distearoyl-sn-glycero-3-phosphocholine; cholesterol; 1,2-distearoyl-sn-glycero-3-phosphoethanolamine-N-[methoxy(polyethylene glycol)-2000] and cytotoxic ceramide analog, in a mol% ranging from 37 mol% to 5 mol%, 19 mol% to 2 mol%, 9 mol% to 31 mol%, 7 mol% to 4 mol% and from 19 mol% to 28 mol%, respectively.

8. The delivery system of claim 1, wherein intracellular triggered release of the agent is a function of the pH value of the target microenvironment and wherein the agent is released through the support destabilization in an acidic environment.

9. The delivery system of claim 8 wherein the acidic pH environment comprises the endosome compartment of said tumor cells.

10. The delivery system of claim 1 suitable for intravenous administration.

11. The delivery system of claim 1, wherein the liposome comprises one or two or three or more of: fully hydrogenated soy phosphatidylcholine, methoxy-polyethylene glycol phosphatidylethanolamine, maleimide-polyethylene glycol phosphatidylethanolamine, N-methylpalmitoyloleoylphosphatidylcholine, phosphatidylserine, phosphatidylcholine, palmitoyloleoylphosphatidylcholine, dipalmitoylphosphatidylcholine, sphingomyelin, distearoylphosphatidylcholine, diphytanoylphosphatidylcholine, N-acetoyl-D-erythro-sphingosine, N-butyroyl-D-erythro-sphingosine, N-hexanoyl-D-erythro-sphingosine, N-octanoyl-D-erythro-sphingosine, N-decanoyl-D-erythro-sphingosine, N-lauroyl-D-erythro-sphingosine, N-myristoyl-D-erythro-sphingosine, N-palmitoyl-D-erythro-sphingosine,phosphatidylglycerol, dioleoylphosphatidylethanolamine, 1,2-dioleoyl-3-trimethylammonium-propane, N-palmitoyl-sphingosine-1-(succinyl[methoxy(polyethylene glycol)]), L-α-Phosphatidylethanolamine-N-(lissamine rhodamine B sulfonyl), cholesteryl hemisuccinate, or cholesterol.

## Patentansprüche

1. Auf Liganden gerichtetes Abgabesystem, umfassend
einen Zielliganden, der selektiv eine Tumorzelle bindet, die an einen einen Wirkstoff tragenden Träger gebunden ist,
worin besagter Träger ein pH-empfindliches Liposom mit einem im Träger gekapselten, eingefangenen oder interkalierten zytotoxischen Ceramidanalogon ist,
worin der Wirkstoff im Träger gekapseltes Doxorubicin ist,
worin das Molverhältnis von zytotoxischem Ceramidanalogon zu Doxorubicin zwischen 3:1 und 1:2 ist,
worin besagtes Liposom zur pH-abhängigen interzellulären Freisetzung von besagtem zytotoxischem Ceramidanalogon und besagtem Wirkstoff fähig ist und worin das zytotoxische Ceramidanalogon N-Acetoyl-D-erythro-Sphingosin, N-Butyroyl-D-erythro-Sphingosin, N-Hexanoyl-D-erythro-Sphingosin, N-Octanoyl-D-erythro-Sphingosin, N-Decanoyl-D-erythro-Sphingosin, N-Lauroyl-D-erythro-Sphingosin, N-Myristoyl-D-erythro-Sphingosin oder N-Palmitoyl-D-erythro-Sphingosin ist.

2. Abgabesystem nach Anspruch 1, worin das zytotoxische Ceramidanalogon C6-Ceramid ist und das Molverhältnis von C6-Ceramid zu Doxorubicin weniger als 2:1 ist, zwischen 1,5:1 und 1:1,5 ist oder etwa 1:1 ist.

3. Abgabesystem nach Anspruch 1, worin der Zielligand eine Krebsstammzelle bindet.

4. Abgabesystem nach Anspruch 1, worin der Zielligand (i) Nucleolin bindet oder (ii) ein Peptid umfassend die Aminosäuresequenz von SEQ ID NR. 1 ist.

5. Abgabesystem nach Anspruch 1, worin der Zielligand an den Träger durch einen Spacer gebunden ist, der zwischen dem Zielliganden und dem Träger positioniert ist, so dass die Interaktion des Liganden mit dem Ziel nicht behindert ist.

6. Abgabesystem nach Anspruch 1, worin der Träger ein Liposom umfassend 1,2-Dioleoyl-sn-glycero-3-phosphoethanolamin; 3β-Hydroxy-5-cholesten-3-hemisuccinat; 1,2-Distearoyl-sn-glycero-3-phosphocholin; Cholesterin; 1,2-Distearoyl-sn-glycero-3-phosphoethanolamin-N-[methoxy(polyethylenglycol)-2000]; und zytotoxisches Ceramidanalogon ist.

7. Abgabesystem nach Anspruch 1, worin der Träger ein Liposom umfassend 1,2-Dioleoyl-sn-glycero-3-phosphoethanolamin; 3β-Hydroxy-5-cholesten-3-hemisuccinat; 1,2-Distearoyl-sn-glycero-3-phosphocholin; Cholesterin; 1,2-Distearoyl-sn-glycero-3-phosphoethanolamin-N-[methoxy(polyethylenglycol)-2000] und zytotoxisches Ceramidanalogon in einem Mol-%-Bereich von 37 Mol-% bis 5 Mol-%, 19 Mol-% bis 2 Mol-%, 9 Mol-% bis 31 Mol-%, 7 Mol-% bis 4 Mol-% bzw. von 19 Mol-% bis 28 Mol-% ist.

8. Abgabesystem nach Anspruch 1, worin interzelluläre ausgelöste Freisetzung des Wirkstoffs eine Funktion des pH-Wertes der Zielmikroumgebung ist und worin der Wirkstoff durch die Trägerdestabilisierung in einer sauren Umgebung freigesetzt wird.

9. Abgabesystem nach Anspruch 8, worin die saure pH-Umgebung das Endosom-Kompartiment besagter Tumorzellen umfasst.

10. Abgabesystem nach Anspruch 1, das für intravenöse Verabreichung geeignet ist.

11. Abgabesystem nach Anspruch 1, worin das Liposom eines oder zwei oder drei oder mehr von folgenden umfasst: vollständig hydriertes Sojaphosphatidylcholin, Methoxy-polyethylenglycol-phosphatidylethanolamin, Maleimid-polyethylenglycolphosphatidylethanolamin, N-Methylpalmitoyloleoylphosphatidylcholin, Phosphatidylserin, Phosphatidylcholin, Palmitoyloleoylphosphatidylcholin, Dipalmitoylphosphatidylcholin, Sphingomyelin, Distearoylphosphatidylcholin, Diphytanoylphosphatidylcholin, N-Acetoyl-D-erythro-Sphingosin, N-Butyroyl-D-erythro-Sphingosin, N-Hexanoyl-D-erythro-Sphingosin, N-Octanoyl-D-erythro-Sphingosin, N-Decanoyl-D-erythro-Sphingosin, N-Lauroyl-D-erythro-Sphingosin, N-Myristoyl-D-erythro-Sphingosin, N-Palmitoyl-D-erythro-Sphingosin, Phosphatidylglycerol, Dioleoylphosphatidylethanolamin, 1,2-Dioleoyl-3-trimethylammonium-propan, N-Palmitoyl-Sphingosin-1-(succinyl[methoxy(polyethylenglycol)]), L-α-Phosphatidylethanolamin-N-(lissaminrhodamin-B-sulfonyl), Cholesterylhemisuccinat oder Cholesterin.

## Revendications

1. Un système de délivrance ciblé par un ligand comprenant :
un ligand de ciblage qui lie sélectivement une cellule tumorale liée à un support portant un agent,
ledit support étant un liposome sensible au pH ayant un analogue de céramide cytotoxique encapsulé, piégé ou intercalé dans le support,
l'agent étant la doxorubicine encapsulée dans le support,
le rapport molaire de l'analogue de céramide cytotoxique à la doxorubicine étant entre 3:1 et 1:2,
ledit liposome étant capable de la libération intracellulaire pH-dépendante dudit analogue de céramide cytotoxique et dudit agent, et l'analogue de céramide cytotoxique étant la N-acétoyl-D-érythro-sphingosine, la N-butyroyl-D-érythro-sphingosine, la N-hexanoyl-D-érythro- sphingosine, la N-octanoyl-D-érythro-sphingosine, la N-décanoyl-D-érythro-sphingosine, la N-lauroyl-D-érythro-sphingosine, la N-myristoyl-D-érythro-sphingosine, ou la N-palmitoyl-D-érythro-sphingosine.

2. Le système de délivrance de la revendication 1, dans lequel l'analogue de céramide cytotoxique est la C6-Céramide et le rapport molaire de la C6-céramide à la doxorubicine est inférieur à 2:1, est entre 1,5:1 et 1:1,5 ou est environ 1:1.

3. Le système de délivrance de la revendication 1, dans lequel le ligand de ciblage lie une cellule souche de cancer.

4. Le système de délivrance de la revendication 1, dans lequel le ligand de ciblage (i) lie la nucléoline ou (ii) est un peptide comprenant la séquence d'acides aminés de SEQ ID NO: 1.

5. Le système de délivrance de la revendication 1, dans lequel le ligand de ciblage est lié au support par un espaceur positionné entre le ligand de ciblage et le support de manière à ce que l'interaction du ligand avec la cible ne soit pas gênée.

6. Le système de délivrance de la revendication 1, dans lequel le support est un liposome comprenant la 1,2- dioléoyl-sn-glycéro-3-phosphoéthanolamine ; le 3β-hydroxy-5-cholestène-3-hémisuccinate ; la 1,2- distéaroyl-sn-glycéro-3-phosphocholine ; le cholestérol ; la 1,2-distéaroyl-sn-glycéro-3-phosphoéthanolamine-N-[méthoxy(polyéthylène glycol)-2000] ; et un analogue de céramide cytotoxique.

7. Le système de délivrance de la revendication 1, dans lequel le support est un liposome comprenant la 1,2- dioléoyl-sn-glycéro-3-phosphoéthanolamine ; le 3β-hydroxy-5-cholestène-3-hémisuccinate ; la 1,2- distéaroyl-sn-glycéro-3-phosphocholine ; le cholestérol ; la 1,2-distéaroyl-sn-glycéro-3-phosphoéthanolamine-N-[méthoxy(polyéthylène glycol)-2000] et un analogue de céramide cytotoxique en % molaire allant de 37 % mol à 5 % mol, 19 % mol à 2 % mol, 9 % mol à 31 % mol, 7 % mol à 4 % mol et de 19 % mol à 28 % mol, respectivement.

8. Le système de délivrance de la revendication 1, dans lequel la libération déclenchée intracellulaire de l'agent est une fonction de la valeur de pH du microenvironnement cible et dans lequel l'agent est libéré par la déstabilisation du support dans un environnement acide.

9. Le système de délivrance de la revendication 8 dans lequel l'environnement pH acide comprend le compartiment d'endosome desdites cellules tumorales.

10. Le système de délivrance de la revendication 1 convenant à l'administration intraveineuse.

11. Le système de délivrance de la revendication 1, dans lequel le liposome comprend un ou deux ou trois ou plus de : phosphatidylcholine de soja entièrement hydrogénée, méthoxy-polyéthylène glycol phosphatidyléthanolamine, maléimide-polyéthylène glycol phosphatidyléthanolamine, N-méthylpalmitoyloléoylphosphatidylcholine, phosphatidylsérine, phosphatidylcholine, palmitoyloléoylphosphatidylcholine, dipalmitoylphosphatidylcholine, sphingomyéline, distéaroylphosphatidylcholine, diphytanoylphosphatidylcholine, N-acétoyl-D-érythro-sphingosine, N-butyroyl-D-érythro-sphingosine, N-hexanoyl-D-érythro-sphingosine, N-octanoyl-D-érythro-sphingosine, N-décanoyl-D-érythro-sphingosine, N-lauroyl-D-érythro-sphingosine, N-myristoyl-D-érythro-sphingosine, N-palmitoyl-D-érythro-sphingosine, phosphatidylglycérol, dioléoylphosphatidyléthanolamine, 1,2-dioléoyl-3-triméthylammonium-propane, N-palmitoyl-sphingosine-1-(succinyl[méthoxy(polyéthylène glycol)]), L-α-Phosphatidyléthanolamine-N-(lissamine rhodamine B sulfonyl), cholestéryl hémisuccinate ou cholestérol.
